(19) Europäisches Patentamt
European Patent Office
Office européen des brevets

(11) **EP 3 827 248 B1**

(12) **EUROPEAN PATENT SPECIFICATION**

(45) Date of publication and mention
of the grant of the patent:
**12.06.2024 Bulletin 2024/24**

(21) Application number: **19752282.4**

(22) Date of filing: **24.07.2019**

(51) International Patent Classification (IPC):
*G01N 21/64* (2006.01)    *G01N 33/58* (2006.01)

(52) Cooperative Patent Classification (CPC):
**G01N 21/6428; G01N 21/6458;** G01N 33/582;
G01N 2021/6441

(86) International application number:
**PCT/JP2019/029075**

(87) International publication number:
**WO 2020/022394 (30.01.2020 Gazette 2020/05)**

(54) **INFORMATION PROCESSING APPARATUS AND MICROSCOPE FOR SEPARATING THE FLUORESCENCE OF A FLUORESCENT REAGEANT FROM THE AUTOFLUORESCENCE OF A SPECIMEN**

INFORMATIONSVERARBEITUNGSVORRICHTUNG UND MIKROSKOP ZUR TRENNUNG DER FLUORESZENZ EINES FLUORESZENZREAGENZES VON DER AUTOFLUORESZENZ EINER PROBE

APPAREIL DE TRAITEMENT D'INFORMATIONS ET MICROSCOPE POUR SÉPARER LA FLUORESCENCE D'UN RÉACTIF FLUORESCENT DE L'AUTO-FLUORESCENCE D'UN ÉCHANTILLON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.07.2018 JP 2018138662**
**17.07.2019 JP 2019132300**

(43) Date of publication of application:
**02.06.2021 Bulletin 2021/22**

(73) Proprietor: **Sony Group Corporation**
**Tokyo 108-0075 (JP)**

(72) Inventors:
• **NAKAMURA, Tomohiko**
**Tokyo 108-0075 (JP)**
• **NAKAGAWA, Kazuhiro**
**Tokyo 108-0075 (JP)**
• **TATSUTA, Hirokazu**
**Tokyo 108-0075 (JP)**
• **CHUBACHI, Hideya**
**Tokyo 108-0075 (JP)**

(74) Representative: **D Young & Co LLP**
**3 Noble Street**
**London EC2V 7BQ (GB)**

(56) References cited:
WO-A1-2017/072320    US-A1- 2008 294 032
US-A1- 2014 376 087    US-A1- 2016 035 100

• JAMES R. MANSFIELD ET AL: "Autofluorescence removal, multiplexing, and automated analysis methods for in-vivo fluorescence imaging", JOURNAL OF BIOMEDICAL OPTICS, vol. 10, no. 4, 2005, page 041207, XP055269855, ISSN: 1083-3668, DOI: 10.1117/1.2032458
• ANDREW KNIGHT ET AL: "Distinguishing GFP from cellular autofluorescence.", BIOPHOTONICS INTERNATIONAL, vol. 8, September 2001 (2001-09), pages 1-6, XP055158156,

**Description**

[Technical Field]

The present disclosure relates to an information processing apparatus and a microscope system.

[Background Art]

In the past, in immunofluorescence staining, an antigen has been visualized by using a fluorescently labeled secondary antibody. When this method is used, the type of visualizable antigen is limited by the type of antibody used as a primary antibody.

A fluorescence amplification method using an enzyme-labeled secondary antibody is applied to solve the above problem. When this method is used, a large number of antigens are visualized by the repetition of antigen-antibody reaction, depositing of fluorescent pigment into a specimen, and separation of antibody although the types of visualizable antigen are limited by the type of available enzyme as is the case with conventional immunostaining. However, as disclosed in NPL 1, when the fluorescence amplification method is used, the time required for work may increase with an increase in the number of repetitions of a cycle of antigen-antibody reaction to antibody separation. Further, each time the cycle is repeated, the specimen may deteriorate.

A method of applying a reagent labeled with a direct fluorescent pigment to a primary antibody or a fluorescent probe is developed to solve the above problems. This method makes it possible to visualize antigens or target molecules for all types of fluorescent pigments.

[Citation List]

[Non Patent Literature]

[NPL 1]
Zhang W. et al. "Fully automated 5-plex fluorescent immunohistochemistry with tyramide signal amplification and same species antibodies," [online], May 15, 2017, Laboratory Investigation, Internet <URL: http://www.nature.com/articles/labinvest201737>
US 2016/0035100 A1 relates to spatial unmixing in fluorescence microscope, and more particularly, to estimating reference spectra for predominantly broadband regions of an image and using the reference spectra to unmix desired regions of the image.
US 2008/0294032 A1 is directed to algorithms for estimating the pure spectrum of one or more sample components from spectral image cube data, even where one or more of these components are only present in a mixed form.
US 2014/0376087 teaches an image processing apparatus, microscope system, and image processing method for processing a microscope observation image of a biological specimen.
Non-patent document "Autofluorescence removal, multiplexing, and automated analysis methods for in-vivo fluorescence imaging" (Mansfield et al.), Journal of biomedical Optics, vol. 10, no. 4, 1 January 2005, page 041207, ISSN: 1083-3668, DOI: 10.1117/1.2032458, describes the multispectral imaging approach for separating autofluorescence to *in-vivo* imaging.
Non-patent document "Distinguishing GFP from Cellular Autofluorescence" (Knight and Billinton), Biophotonics International, vol. 8, 1 September 2001, pages 1-6, describes a range of photonic techniques available to tackle the problem of endogenous autofluorescence and to allow effective discrimination of GFP from autofluorescence.
WO 2017/072320 A1 relates to the use of mid-infrared (MIR) spectroscopy to assess the quality of tissue samples.

[Summary]

[Technical Problem]

However, when the above method is used, only limited types of fluorescent pigments can be coupled to a primary antibody and a fluorescent probe. In some cases, therefore, a fluorescence signal to be measured may be buried in an autofluorescence signal of a specimen. As a result, it may be difficult to detect such a fluorescence signal.

The present disclosure has been made in view of the above circumstances. An object of the present disclosure is to provide an information processing apparatus, an information processing method, an information processing system, and a program that are novel, improved, and capable of more properly separating at least one of a fluorescence signal to be measured or an autofluorescence signal of a specimen from image information regarding the specimen.

[Solution to Problem]

The invention is defined by the appended claims.

[Advantageous Effects of Invention]

As described above, the present disclosure is able to more properly separate at least one of a fluorescence signal to be measured or an autofluorescence signal of a specimen from image information regarding the specimen.
It should be noted that the advantageous effect described above is not necessarily restrictive. Any of the advantageous effects described in the present specification or other advantageous effects contemplatable from the present specification may be provided in addition to or in place of the above-described advantageous effect.

[Brief Description of Drawings]

[Fig. 1] Fig. 1 is a diagram illustrating the removal of an autofluorescence signal derived from a specimen 20.
[Fig. 2] Fig. 2 is another diagram illustrating the removal of the autofluorescence signal derived from the specimen 20.
[Fig. 3] Fig. 3 is a block diagram illustrating an exemplary configuration of an information processing system according to an embodiment of the present disclosure.
[Fig. 4] Fig. 4 is a flowchart illustrating various exemplary processes performed by an information processing apparatus 100.
[Fig. 5] Fig. 5 is another flowchart illustrating various exemplary processes performed by the information processing apparatus 100.
[fig.6]Fig. 6 is a diagram illustrating a practical example of the removal of the autofluorescence signal derived from the specimen 20.
[fig.7]Fig. 7 is another diagram illustrating a practical example of the removal of the autofluorescence signal derived from the specimen 20.
[fig.8A]Fig. 8A is another diagram illustrating a practical example of the removal of the autofluorescence signal derived from the specimen 20.
[fig.8B]Fig. 8B is another diagram illustrating a practical example of the removal of the autofluorescence signal derived from the specimen 20.
[fig.9A]Fig. 9A is another diagram illustrating a practical example of the removal of the autofluorescence signal derived from the specimen 20.
[fig.9B]Fig. 9B is another diagram illustrating a practical example of the removal of the autofluorescence signal derived from the specimen 20.
[fig.10]Fig. 10 is a diagram illustrating an example of image information when a tonsil tissue is used as the specimen 20 and an autofluorescence signal obtained from the tonsil tissue is used as an autofluorescence signal used in color separation calculation when a fluorescence signal is extracted from image information.
[fig.11]Fig. 11 is a diagram illustrating an example of image information when a tonsil tissue is used as the specimen 20 and an autofluorescence signal obtained from the lung tissue that is a tissue different from the tonsil tissue is used as an autofluorescence signal used in color separation calculation when a fluorescence signal is extracted from image information.
[fig.12]Fig. 12 is a diagram illustrating a color separation result when a marker antibody CD4 labeled with a fluorescence dye Alexa Fluor 488 is used as a fluorescent reagent 10.
[fig.13]Fig. 13 is a diagram illustrating a color separation result when a marker antibody PDL-1 labeled with a fluorescence dye Alexa Fluor 594 is used as the fluorescent reagent 10.
[fig.14]Fig. 14 is a diagram illustrating a practical example of the analysis of the specimen 20 in an immobilized state.
[fig.15]Fig. 15 is another diagram illustrating a practical example of the analysis of the specimen 20 in the immobilized state.
[fig.16] Fig. 16 is another diagram illustrating a practical example of the analysis of the specimen 20 in the immobilized state.
[fig.17]Fig. 17 is another diagram illustrating a practical example of the analysis of the specimen 20 in the immobilized state.
[fig.18]Fig. 18 is a block diagram illustrating an exemplary hardware configuration of the information processing apparatus 100.

[fig.19]Fig. 19 is a diagram illustrating how actual specimen information and reagent information differ from catalog values and literature values.

[fig.20]Fig. 20 is another diagram illustrating how actual specimen information and reagent information differ from catalog values and literature values.

[fig.21]Fig. 21 is a block diagram depicting an exemplary configuration of an information processing system according to a modification of the present embodiment.

[fig.22]Fig. 22 is a block diagram depicting an example of a schematic configuration of a diagnostic system.

**Description of Embodiments**

A preferred embodiment of the present disclosure will now be described in detail with reference to the accompanying drawings. In the present specification and the accompanying drawings, elements having substantially the same functions are designated by the same reference numerals and will not be redundantly described.

The description will be given in the following order.

1. Overview
2. Embodiment
3. Practical Examples
4. Exemplary Hardware Configuration
5. Remarks
6. Modification of System Configuration
7. Conclusions
8. Exemplary Application

<1. Overview>

First of all, the present disclosure is outlined below.

In conventional immunostaining, for example, an antigen recognized by a primary antibody has been visualized by depositing a coloring pigment (color absorbing pigment) into a specimen through the use of an enzyme-labeled secondary antibody. When this method is used to visualize a large number of antigens, the types of available enzyme are limited. Further, the types of visualizable antigens are also limited depending on the resolution of a pigment spectrum.

Meanwhile, in the past, in immunofluorescence staining, an antigen has been visualized by using a fluorescently labeled secondary antibody. When this method is used, the types of visualizable antigens are limited depending on the type of antibody used as a primary antibody.

In order to solve the above problem, a fluorescence amplification method is exercised by using an enzyme-labeled secondary antibody. When this method is used, a large number of antigens are visualized by the repetition of antigen-antibody reaction, depositing of fluorescent pigment into a specimen, and separation of antibody although the types of visualizable antigen are limited by the type of available enzyme as is the case with conventional immunofluorescence staining. However, as disclosed in NPL 1, when the fluorescence amplification method is used, the time required for work may increase with an increase in the number of repetitions of a cycle of antigen-antibody reaction to antibody separation. Further, each time the cycle is repeated, the specimen may deteriorate.

In order to solve the above problems, a method of applying a reagent labeled with a direct fluorescent pigment to a primary antibody or a fluorescent probe is developed. This method makes it possible to visualize antigens or target molecules for all types of fluorescent pigments. However, this method is such that only limited types of fluorescent pigments can be coupled to a primary antibody and a fluorescent probe. In some cases, therefore, a fluorescence signal to be measured may be buried in an autofluorescence signal of a specimen. As a result, it may be difficult to detect such a fluorescence signal.

Particularly, in a case where a tissue sample is used, a difference in autofluorescent components occurs between internal organs, and a difference in autofluorescence intensity is caused by an immobilized state of the tissue sample. This makes it more difficult to distinguish between the fluorescence signal to be measured and the autofluorescence signal of the specimen.

In conventional measurements made on a tissue sample after fluorescent labeling, in order to remove the autofluorescence signal derived from a specimen, for example, the fluorescence signal of an unlabeled specimen having the same derivation as or similar to the former specimen is measured, and then the measured fluorescence signal of the unlabeled specimen is subtracted from the fluorescence signal acquired from the tissue sample. However, the spectrum of autofluorescence and the spatial distribution of the spectrum differ depending, for example, on the state of the specimen, the method of specimen storage, and the method of specimen preparation. Therefore, in a case where this method is used, it is necessary in each measurement to measure the fluorescence signal of the unlabeled specimen and perform a subtraction process by using the fluorescence signal. Consequently, a heavy burden is imposed on a practitioner.

In view of the above circumstances, the inventors of the present disclosure have created a technology according to the present disclosure. The inventors of the present disclosure have worked out the technology according to the present disclosure based on a finding that the spectrum of autofluorescent components included in an autofluorescence spectrum remains unvaried while the overall autofluorescence spectrum of a specimen varies with the method of specimen immobilization, and that it is the fluorescence signal component ratio of each component that matters.

More specifically, based on information regarding a specimen and information regarding a fluorescent reagent, an information processing apparatus 100 according to the present disclosure separates the autofluorescence signal of the specimen and the fluorescence signal of the fluorescent reagent from image information regarding the specimen stained by at least one fluorescent reagent. Further, in a case where the specimen is stained by two or more fluorescent reagents, the information processing apparatus 100 separates the fluorescence signal of each fluorescent reagent from the image information in accordance with the information regarding the specimen and the information regarding the fluorescent reagent.

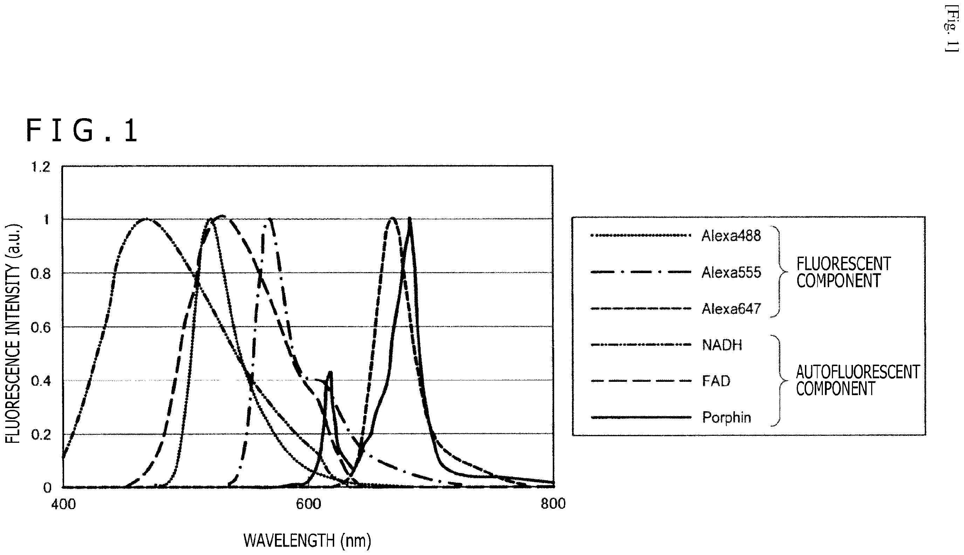

Here, the "information regarding the specimen" (hereinafter conveniently referred to as the "specimen information") is information including measurement channel and spectral information unique to autofluorescent components included in the specimen. The "measurement channel" is a concept representing one or more elements included in the autofluorescence signal. More specifically, the autofluorescence spectrum is a mixture of fluorescence spectra possessed by one or more autofluorescent components. For example, Fig. 1 illustrates the spectra of fluorescence including autofluorescence in cases where Alexa Fluor 488 (designated as "Alexa 488" in Fig. 1), Alexa Fluor 555 (designated as "Alexa 555" in Fig. 1), and Alexa Fluor 647 (designated as "Alexa 647" in Fig. 1) are used as the fluorescent reagent. Included as the autofluorescent components are NADH (nicotinamide adenine dinucleotide reduced form), FAD (flavin adenine dinucleotide), and Porphin. A mixture of fluorescence spectra possessed by these autofluorescent components is included in Fig. 1 as the autofluorescence spectrum. In the example of Fig. 1, the measurement channel according to the present disclosure is a concept indicative of NADH, FAD, and Porphin, which are included in the autofluorescence spectrum, and a total of three measurement channels are used (i.e., the measurement channel according to the present disclosure is information indicative of an autofluorescent component included in the specimen). As the number of autofluorescent components varies from one specimen to another, the measurement channel is associated with each specimen and managed as the specimen information. Further, the "spectral information" included in the specimen information is information regarding an autofluorescence spectrum possessed by each autofluorescent component included in each specimen. It should be noted that the contents of the specimen information are not limited to those described above. The specimen information will be described in detail later.

The "information regarding the fluorescent reagent" (hereinafter conveniently referred to as the "reagent information") is information including spectral information regarding fluorescent components included in the fluorescent reagent. The "spectral information" included in the reagent information is information regarding a fluorescence spectrum possessed by each fluorescent component included in each fluorescent reagent. It should be noted that the contents of the reagent information are not limited to those described above. The reagent information will be described in detail later.

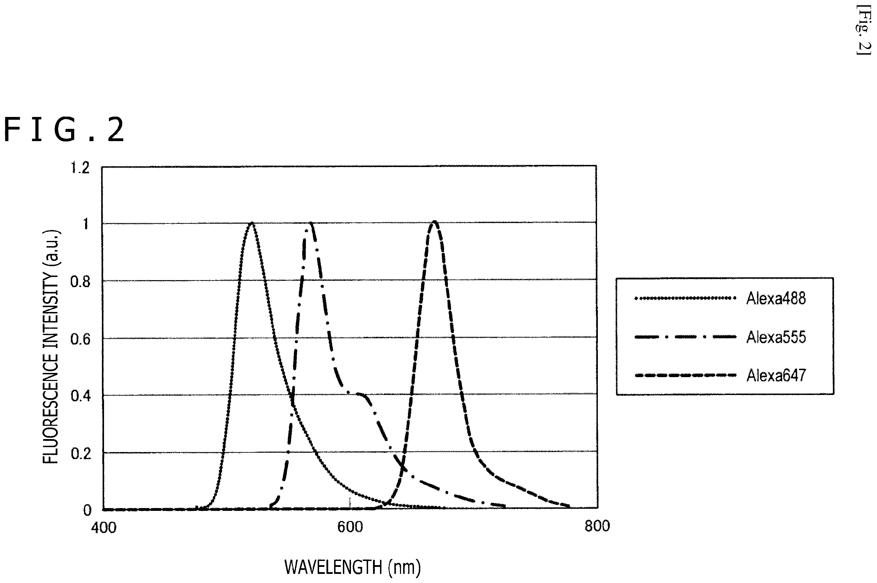

The information processing apparatus 100 according to the present disclosure is able to extract the fluorescence signal of the fluorescent reagent by estimating the autofluorescence signal derived from the specimen in accordance with the measurement channel and spectral information included in the specimen information and with the spectral information included in the reagent information, and removing the autofluorescence signal from the image information. For example, the information processing apparatus 100 is able to extract fluorescence signals provided by Alexa Fluor 488, Alexa Fluor 555, and Alexa Fluor 647 as indicated in Fig. 2 by removing, from the image information, the autofluorescence signals provided by NADH, FAD, and Porphin, which are the autofluorescent components.

The information processing apparatus 100 according to the present disclosure need not measure the fluorescence signal of an unlabeled specimen for the purpose of performing the subtraction process on the autofluorescence signal as described above. This reduces the burden on the practitioner. Further, the information processing apparatus 100 is able to separate the autofluorescence signal and the fluorescence signal with higher accuracy by using both the specimen information and the reagent information instead of using either one of the specimen information and the reagent information.

Furthermore, in order to acquire the fluorescence signal to be measured, the method of amplifying the fluorescence signal until the autofluorescence signal derived from a specimen is negligible has been conventionally and widely used. However, when this method is used, it is difficult to acquire the autofluorescence signal derived from a specimen. Meanwhile, the information processing apparatus 100 according to the present disclosure is able to extract the specimen-derived autofluorescence signal from the image information. Consequently, based on the distribution of the autofluorescence signal and fluorescence signal in the image information, the information processing apparatus 100 is able to perform segmentation (or region segmentation) for recognizing a region of an object (e.g., cell, intracellular structure (cytoplasm, cell membrane, or nucleus), or tissue (tumor part, non-tumor part, connective tissue, blood vessel, vascular wall, lymphatic vessel, fibrosis structure, or necrosis)) included in the image information.

Moreover, as the overall autofluorescence signal of a specimen varies with the immobilized state of the specimen (e.g., immobilization method), the information processing apparatus 100 is able to analyze (evaluate) the immobilized state of the specimen in accordance with the extracted autofluorescence signal. More specifically, the information processing apparatus 100 is able to analyze the immobilized state of the specimen in accordance with the autofluorescence signal component ratio of each of two or more autofluorescence-emitting components.

<2. Embodiment>

The present disclosure has been outlined above. An embodiment of the present disclosure will now be described.

(2.1. Exemplary Configuration)

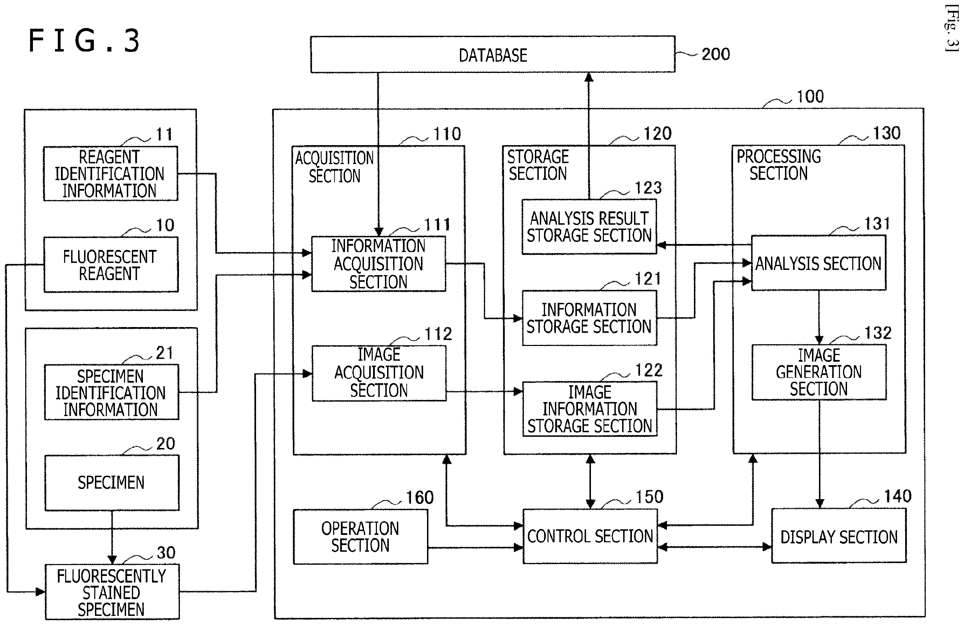

First of all, an exemplary configuration of an information processing system according to the present embodiment will be described with reference to Fig. 3. As illustrated in Fig. 3, the information processing system according to the present embodiment includes an information processing apparatus 100 and a database 200. A fluorescent reagent 10, a specimen 20, and a fluorescently stained specimen 30 exist as the input to the information processing system.

(Fluorescent Reagent 10)

The fluorescent reagent 10 is a chemical used to stain the specimen 20. The fluorescent reagent 10 is, for example, a fluorescent antibody (including a primary antibody used for direct labeling or a secondary antibody used for indirect labeling), a fluorescent probe, or a nuclear staining reagent. However, the fluorescent reagent 10 is not limited to such types. Further, identification information capable of identifying the fluorescent reagent 10 (and the production lot of the fluorescent reagent 10) (hereinafter referred to as the "reagent identification information 11") is attached to the fluorescent reagent 10 for management purposes. The reagent identification information 11 is, for example, barcode information (e.g., one-dimensional barcode information and two-dimensional barcode information). However, the reagent identification information 11 is not limited to such information. Even when the fluorescent reagent 10 is produced as the same product (the same type of product), its properties vary from one production lot to another depending, for example, on the production method and the state of a cell at the time of antibody acquisition. The fluorescent reagent 10 varies from one production lot to another in terms, for example, of spectral information, quantum yield, or fluorescent labeling ratio (referred to also as the F/P value or the fluorescein/protein value and indicative of the number of fluorescent molecules for antibody labeling). Therefore, in the information processing system according to the present embodiment, the reagent identification information 11 is attached to the fluorescent reagent 10 in order to manage the fluorescent reagent 10 on an individual production lot basis (i.e., the reagent information regarding each fluorescent reagent 10 is managed on an individual production lot basis). This enables the information processing apparatus 100 to separate the fluorescence signal and the autofluorescence signal in additional consideration of slight property differences between the production lots. It should be noted that managing the fluorescent reagent 10 on an individual production lot basis is merely an example. The fluorescent reagent 10 may alternatively be managed in units smaller than a production lot.

(Specimen 20)

The specimen 20 is prepared from a human specimen or tissue sample taken from a human body for the purpose, for example, of pathological diagnosis or clinical examination. The specimen 20 is not limited to a particular type of sampled tissue (e.g., internal organ or cell), target disease, target person's attributes (e.g., age, gender, blood type, or race), or target person's lifestyle (e.g., eating habits, exercise habits, or smoking habits). Further, identification information capable of identifying each specimen 20 (hereinafter referred to as the "specimen identification information 21") is attached to the specimen 20 for specimen management purposes. As is the case with the reagent identification information 11, the specimen identification information 21 is, for example, barcode information (e.g., one-dimensional barcode information and two-dimensional barcode information). However, the specimen identification information 21 is not limited to such information. The properties of the specimen 20 vary, for example, with the type of sampled tissue, the type of target disease, target person's attributes, or target person's lifestyle. As regards the specimen 20, for example, the measurement channel or the spectral information varies, for instance, with the type of sampled tissue. Accordingly, in the information processing system according to the present embodiment, the specimen identification information 21 is attached to the specimen 20 in order to manage the specimen 20 on an individual basis. This enables the information processing apparatus 100 to separate the fluorescence signal and the autofluorescence signal in additional consideration of slight property differences between each specimen 20.

(Fluorescently Stained Specimen 30)

The fluorescently stained specimen 30 is prepared by allowing the fluorescent reagent 10 to stain the specimen 20. The present embodiment assumes that the fluorescently stained specimen 30 is prepared by allowing at least one fluorescent reagent 10 to stain the specimen 20. However, the number of fluorescent reagents 10 used for staining is not particularly limited. Further, the method of staining is determined in accordance, for example, with the combination of the specimen 20 and fluorescent reagent 10, and is not particularly limited. The fluorescently stained specimen 30 is inputted to the information processing apparatus 100 and then imaged.

(Information Processing Apparatus 100)

As illustrated in Fig. 3, the information processing apparatus 100 includes an acquisition section 110, a storage section 120, a processing section 130, a display section 140, a control section 150, and an operation section 160.

(Acquisition Section 110)

The acquisition section 110 acquires information that is to be used in various processes of the information processing apparatus 100. As illustrated in Fig. 3, the acquisition section 110 includes an information acquisition section 111 and an image acquisition section 112.

(Information Acquisition Section 111)

The information acquisition section 111 acquires the reagent information and the specimen information. More specifically, the information acquisition section 111 acquires the reagent identification information 11, which is attached to the fluorescent reagent 10 used for preparing the fluorescently stained specimen 30, and the specimen identification information 21, which is attached to the specimen 20. The information acquisition section 111 uses, for example, a barcode reader to acquire the reagent identification information 11 and the specimen identification information 21. Then, the information acquisition section 111 acquires, from a database 200, the reagent information based on the reagent identification information 11 and the specimen information based on the specimen identification information 21. The information acquisition section 111 stores the acquired information in a later-described information storage section 121.

(Image Acquisition Section 112)

The image acquisition section 112 acquires the image information regarding the fluorescently stained specimen 30 (the specimen 20 stained by at least one fluorescent reagent 10). More specifically, the image acquisition section 112 includes a predetermined imaging element (e.g., a CCD or CMOS sensor), and acquires the image information by imaging the fluorescently stained specimen 30 with the imaging element. Here, it should be noted that the "image information" is a concept including not only an image of the fluorescently stained specimen 30 but also a measured or other value not visualized as an image. For example, the image information may include information regarding a wavelength of spectrum of fluorescence (hereinafter referred to as fluorescence spectrum) radiated from the fluorescently stained specimen 30. The image acquisition section 112 stores the image information in a later-described image information storage section 122.

(Storage Section 120)

The storage section 120 stores (memorizes) information that is to be used in various processes of the information processing apparatus 100 or is outputted by the various processes. As illustrated in Fig. 3, the storage section 120 includes an information storage section 121, an image information storage section 122, and an analysis result storage section 123.

(Information Storage Section 121)

The information storage section 121 stores the reagent information and specimen information acquired by the information acquisition section 111. It should be noted that, after termination of an analysis process performed by a later-described analysis section 131 and an image information generation process (image information reconstruction process) performed by a later-described image generation section 132, the information storage section 121 may increase its available capacity by deleting the reagent information and specimen information used for processing.

(Image Information Storage Section 122)

The image information storage section 122 stores the image information regarding the fluorescently stained specimen 30 acquired by the image acquisition section 112. As is the case with the information storage section 121, it should be noted that, after termination of the analysis process performed by the analysis section 131 and the image information generation process (image information reconstruction process) performed by the image generation section 132, the image information storage section 122 may increase its available capacity by deleting the image information used for processing.

(Analysis Result Storage Section 123)

The analysis result storage section 123 stores the result of the analysis process performed by the later-described analysis section 131. The analysis result storage section 123 stores, for example, the fluorescence signal of the fluorescent reagent or the autofluorescence signal of the specimen 20, which are separated by the analysis section 131. Further, the analysis result storage section 123 supplies the result of the analysis process to the database 200 in order to provide increased analysis accuracy, for example, through separate machine learning. It should be noted that, after supplying the result of the analysis process to the database 200, the analysis result storage section 123 may increase its available capacity by appropriately deleting its stored result of the analysis process.

(Processing Section 130)

The processing section 130 has a function of performing various processes by using the image information, the reagent information, and the specimen information. As illustrated in Fig. 3, the processing section 130 includes the analysis section 131 and the image generation section 132.

(Analysis Section 131)

The analysis section 131 performs various analysis processes by using the image information, the specimen information, and the reagent information. The analysis section 131 performs, for example, a process of separating the autofluorescence signal of the specimen 20 and the fluorescence signal of the fluorescent reagent 10 from the image information in accordance with specimen information and the reagent information.
More specifically, the analysis section 131 recognizes one or more elements of the autofluorescence signal in accordance with the measurement channel included in the specimen information. The analysis section 131 recognizes, for example, one or more autofluorescent components of the autofluorescence signal. The analysis section 131 then predicts the autofluorescence signal included in the image information by using the spectral information regarding the autofluorescent components included in the specimen information. Subsequently, based on the spectral information regarding the fluorescent components of the fluorescent reagent 10, which is included in the reagent information, and on the predicted autofluorescence signal, the analysis section 131 separates the autofluorescence signal and fluorescence signal from the image information.
Here, in a case where the specimen 20 is stained by two or more fluorescent reagents 10, the analysis section 131 separates the fluorescence signal of each of the two or more fluorescent reagents 10 from the image information (or from the fluorescence signal separated together with the autofluorescence signal) in accordance with the specimen information and the reagent information. For example, the analysis section 131 uses the spectral information regarding the fluorescent components of each fluorescent reagent 10, which is included in the reagent information, in order to separate the fluorescence signal of each fluorescent reagent 10 from the whole fluorescence signal after it is separated together with the autofluorescence signal.
Further, in a case where the autofluorescence signal includes two or more autofluorescent components, the analysis section 131 separates the autofluorescence signal of each autofluorescent component from the image information (or from the autofluorescence signal separated together with the fluorescence signal) in accordance with the specimen information and the reagent information. For example, the analysis section 131 uses the spectral information regarding each autofluorescent component, which is included in the specimen information, in order to separate the autofluorescence signal of each autofluorescent component from the whole autofluorescence signal after it is separated together with the fluorescence signal.
After separating the fluorescence signal and the autofluorescence signal, the analysis section 131 uses these signals to perform various processes. For example, the analysis section 131 may use the separated autofluorescence signal to perform a subtraction process (referred to also as the "background subtraction process") on the image information regarding a different specimen 20 in order to extract the fluorescence signal from the image information regarding the different specimen 20. In a case where there exist a plurality of specimens 20 identical or similar in terms, for example, of the tissue used as a specimen 20, the type of a target disease, target person's attributes, or target person's lifestyle, it is highly probable that the autofluorescence signals of such specimens 20 are similar to each other. The similar specimens here include, for example, a tissue slice before staining of a tissue slice (hereinafter referred to as slice) to be stained, a slice neighboring with a stained slice, a slice different from the stained slice in a same block (sampled from a same place as that of the stained slice), a slice in a different block of the same tissue (sampled from a place different from the stained slice)), a slice collected from a different patient or the like. Therefore, in a case where an autofluorescence signal is extracted from a certain specimen 20, the analysis section 131 may extract the fluorescence signal from the image information regarding a different specimen 20 by removing the autofluorescence signal from the image information regarding the different specimen 20. Further, when calculating an S/N value by using the image information regarding the different specimen 20, the analysis section 131 is able to improve the S/N value by using a background after the removal of the autofluorescence signal.

Moreover, the analysis section 131 is able to perform not only the background subtraction process but also various other processes by using the separated fluorescence signal or autofluorescence signal. For example, the analysis section 131 is able to analyze the immobilized state of the specimen 20 by using the above-mentioned signals and perform segmentation (or region segmentation) for recognizing a region of an object (e.g., cell, intracellular structure (cytoplasm, cell membrane, or nucleus), or tissue (tumor part, non-tumor part, connective tissue, blood vessel, vascular wall, lymphatic vessel, fibrosis structure, or necrosis)) included in the image information. The analysis of the immobilized state of the specimen 20 and the segmentation will be described in detail later.

(Image Generation Section 132)

The image generation section 132 generates (reconstructs) image information in accordance with the fluorescence signal or autofluorescence signal separated by the analysis section 131. For example, the image generation section 132 is able to generate image information including only the fluorescence signal and image information including only the autofluorescence signal. If, in such a case, the fluorescence signal includes a plurality of fluorescent components or the autofluorescence signal includes a plurality of autofluorescent components, the image generation section 132 is able to generate the image information on an individual component basis. Further, in a case where the analysis section 131 performs various processes (e.g., the analysis of the immobilized state of the specimen 20, the segmentation, or the calculation of the S/N value) by using the separated fluorescence signal or autofluorescence signal, the image generation section 132 may generate the image information indicative of the result of such processing. According to the present configuration, distribution information of the fluorescent reagent 10 labeled to a target molecule or the like, that is, a secondary spread or intensity, wavelength and positional relationship of fluorescence, is visualized, and the visibility of a doctor or a researcher who is a user can be improved especially in a tissue image analysis region in which information of the target substance is complicated.

The image generation section 132 may control so as to distinguish a fluorescence signal from an autofluorescence signal based on the fluorescence signal or the autofluorescence signal separated by the analysis section 131 to generate image information. In particular, the image generation section 132 may generate image information by controlling improving the luminance of the fluorescence spectrum of the fluorescent reagent 10 labeled to the target molecule or the like, extracting only the fluorescence spectrum of the labeled fluorescent reagent 10 and discoloring the fluorescent reagent 10, extracting a fluorescence spectrum of each of two or more fluorescent reagents 10 from the specimen 20 labeled with the two or more fluorescent reagents 10 and discoloring the fluorescent reagents 10 with different colors, extracting only the autofluorescence spectrum of the specimen 20 and applying division or subtraction, improving the dynamic range or the like. This makes it possible for the user to distinctly distinguish color information originating from the fluorescent reagent coupled to the intended target substance.

(Display Section 140)

The display section 140 presents the image information generated by the image generation section 132 to a user by displaying the image information on a display. It should be noted that the type of display to be used as the display section 140 is not particularly limited. Further, although details are not described in conjunction with the present embodiment, the image information generated by the image generation section 132 may be presented to the user by allowing a projector to project the image information or by allowing a printer to print the image information (i.e., the method of outputting the image information is not particularly limited).

(Control Section 150)

The control section 150 has a function of providing integrated control of all processes performed by the information processing apparatus 100. For example, based on a user's operation input through the operation section 160, the control section 150 controls the start and end of the above-described various processes (e.g., an imaging process on the fluorescently stained specimen 30, an analysis process, an image information generation process (image information reconstruction process), and an image information display process). It should be noted that details of control provided by the control section 150 are not particularly limited. The control section 150 may control, for example, processes commonly performed by a general-purpose computer, a PC, or a tablet PC (e.g., processes concerning an OS (Operating System)).

(Operation Section 160)

The operation section 160 receives an operation input from the user. More specifically, the operation section 160 includes various input means such as a keyboard, a mouse, a button, a touch panel, or a microphone, and allows the user to make various inputs to the information processing apparatus 100 by operating these input means. Information regarding the operation inputs through the operation section 160 is supplied to the control section 150.

(Database 200)

The database 200 is an apparatus that manages the specimen information, the reagent information, and the result of an analysis process. More specifically, the database 200 exercises management by associating the specimen identification information 21 with the specimen information and associating the reagent identification information 11 with the reagent information. This enables the information acquisition section 111 to acquire, from the database 200, the specimen information based on the specimen identification information 21 regarding the specimen 20 to be measured and the reagent information based on the reagent identification information 11 regarding the fluorescent reagent 10.

As described earlier, the specimen information managed by the database 200 includes the measurement channel and spectral information that are unique to an autofluorescent component included in the specimen 20. However, in addition to the above information, the specimen information may also include target information regarding each specimen 20, particularly, information regarding the type of sampled tissue (e.g., internal organ, cell, blood, body fluid, ascites, pleural effusion, etc.), the type of target disease, target person's attributes (e.g., age, gender, blood type, or race), or target person's lifestyle (e.g., eating habits, exercise habits, or smoking habits), and the information including the measurement channel and spectrum information unique to the autofluorescent component included in the specimen 20 and the target information may be associated with each other for each specimen. This makes it possible to easily follow the information including the measurement channel and vector information unique to the autofluorescent component included in the specimen 20 from the target information. For example, it becomes possible for the analysis section to execute a separation process similar to that performed in the past from similarity of target information among a plurality of specimens 20 to reduce the measurement time. It is to be noted that the "tissue used" is not particularly limited to the tissue collected from the target and may include in vivo tissues and cell lines of people and animals and solution, solvent, solute and material included in the target article of the measurement.

Further, the reagent information managed by the database 200 includes the spectral information regarding the fluorescent reagent 10, as described earlier. However, in addition to the above information, the reagent information may also include various other information regarding the fluorescent reagent 10, such as the information regarding a production lot, a fluorescent component, an antibody, a clone, a fluorescent labeling ratio, a quantum yield, a fading coefficient (information indicative of the ease of reducing the fluorescence intensity of the fluorescent reagent 10), and an absorption cross section (or a molar absorption coefficient). Further, the specimen information and the reagent information managed by the database 200 may be managed by configurations different from each other, and information especially regarding reagents may be a reagent database that presents an optimum combination of reagents to the user.

Here, it is assumed that the specimen information and the reagent information are supplied, for example, from a manufacturer (maker) or uniquely measured in the information processing system according to the present disclosure. In many cases, the manufacturers of the fluorescent reagent 10 do not measure and supply, for example, the spectral information and fluorescent labeling ratio regarding each production lot. Therefore, the accuracy of separation of the fluorescence signal and autofluorescence signal can be improved by uniquely measuring and managing the above information in the information processing system according to the present disclosure. Further, for simplification of management, the database 200 may use, for example, catalog values published by the manufacturers (makers) or literature values described in various documents as the specimen information and the reagent information (particularly as the reagent information). However, actual specimen information and reagent information generally differ from catalog values and literature values in many cases. It is therefore preferable that the specimen information and the reagent information be uniquely measured and managed in the information processing system according to the present disclosure as described above.

Further, the accuracy of an analysis process (e.g., the process of separating the fluorescence signal and the autofluorescence signal) can be improved, for example, by a machine learning technology that uses the specimen information, the reagent information, and the result of an analysis process, which are managed by the database 200. Although an agent using, for example, the machine learning technology for learning purposes is not particularly limited, the present embodiment is described below with reference to an exemplary case where learning is performed by the analysis section 131 of the information processing apparatus 100. For example, the analysis section 131 uses a neural network to generate a classifier or estimator that is machine-learned by using learning data, which is obtained by associating the separated fluorescence signal and autofluorescence signal with the image information, specimen information, and reagent information used for separation. Subsequently, in a case where the image information, the specimen information, and the reagent information are newly acquired, the analysis section 131 is able to predict and output the fluorescence signal and autofluorescence signal included in the image information by inputting the newly acquired information to the classifier or the estimator.

Furthermore, the analysis section 131 may output a method of calculating previously performed similar separation processes (separation processes using similar image information, specimen information or reagent information), which are more accurate than the predicted fluorescence signal and autofluorescence signal, statistically or recursively analyzing the details of the calculated processes (e.g., information and parameters used for processing), and improving the process of separating the fluorescence signal and the autofluorescence signal in accordance with the result of analysis. It should be noted that the method of machine learning is not limited to the above-described one. A well-known machine learning technology may be used. Moreover, artificial intelligence may be used to perform the process of separating the fluorescence signal and the autofluorescence signal. Additionally, a machine learning or other technology may be used to improve not only the process of separating the fluorescence signal and the autofluorescence signal, but also various other processes using the separated fluorescence signal and autofluorescence signal (e.g., the analysis of the immobilized state of the specimen 20 or the segmentation).

An exemplary configuration of the information processing system according to the present embodiment has been described above. It should be noted that the configuration described above with reference to Fig. 3 is merely an example. The configuration of the information processing system according to the present embodiment is not limited to the above-described one. For example, the information processing apparatus 100 need not always include all the functional elements depicted in Fig. 3. Further, the information processing apparatus 100 may incorporate the database 200. The functional elements of the information processing apparatus 100 may be flexibly changed based on specifications and operations.

Further, the information processing apparatus 100 may perform a process other than the above-described ones. For example, as far as the reagent information includes information regarding the fluorescent reagent 10, such as the quantum yield, the fluorescent labeling ratio, and the absorption cross section (or molar absorption coefficient), the information processing apparatus 100 may calculate the number of fluorescent molecules in the image information and the number of antibodies coupled to the fluorescent molecules by using the reagent information and the image information from which the autofluorescence signal is removed. More specifically, in a case where an imaging element (e.g., CMOS sensor) performs an imaging process on the fluorescently stained specimen 30, the number of fluorescent molecules in a certain pixel A is expressed by Equation 1 or Equation 2 below. Further, the number of absorbed photons in Equation 2 is expressed by Equation 3 below.

[Math.1]

$$\text{Number of fluorescent molecules of pixel A}$$
$$= \frac{\text{number of photons detected by pixel A}}{\text{number of emitted photons per molecule}}$$
$$\cdots \text{(Equation 1)}$$

[Math.2]

$$\text{Number of emitted photons per molecule}$$
$$= \text{number of absorbed photons} \times \text{quantum yield}$$
$$\cdots \text{(Equation 2)}$$

[Math.3]

$$\text{Number of absorbed photons}$$

$$= \text{excited photon density} \times \text{absorption cross section}$$

$$\cdot \ \cdot \ \cdot \ (\text{Equation 3})$$

The information processing apparatus 100 calculates the number of absorbed photons by actually measuring the excited photon density in Equation 3 and multiplying the excited photon density by the absorption cross section. Further, the information processing apparatus 100 calculates the number of emitted photons per molecule by multiplying the number of absorbed photons by the quantum yield in the reagent information in accordance with Equation 2. The information processing apparatus 100 then calculates the number of fluorescent molecules in the pixel A by dividing the number of photons detected by the pixel A by the number of emitted photons per molecule in accordance with Equation 1. Subsequently, the information processing apparatus 100 is able to calculate the number of fluorescent molecules in each pixel by performing the above computations on all pixels.

Moreover, the information processing apparatus 100 is able to convert the number of fluorescent molecules detected by the pixel A to the number of antibodies by performing the computation expressed by Equation 4 through the use of the fluorescent labeling ratio in the reagent information. Subsequently, the information processing apparatus 100 is able to calculate the number of antibodies in each pixel by performing the computation expressed by Equation 4 on all pixels.

[Math.4]

$$\text{Number of antibodies in pixel A}$$

$$= \frac{\text{number of fluorescent molecules in pixel A}}{\text{fluorescent labeling ratio}}$$

$$\cdot \ \cdot \ \cdot \ (\text{Equation 4})$$

It should be noted that when performing a process of calculating the number of fluorescent molecules and the number of antibodies, the information processing apparatus 100 may correct brightness in the image information to unfaded brightness as far as the reagent information includes the fading coefficient indicative of the ease of reducing the fluorescence intensity. This enables the information processing apparatus 100 to more accurately calculate the number of fluorescent molecules and the number of antibodies in consideration of the influence of a faded fluorescent component. The process of calculating the number of fluorescent molecules and the number of antibodies can be implemented by the analysis section 131 of the information processing apparatus 100. However, such a calculation process is not limited to the above-described one. The information processing apparatus 100 may present, to the user, for example, image information reflecting the information regarding the calculated number of fluorescent molecules and antibodies.

(2.2. Processing Flow)

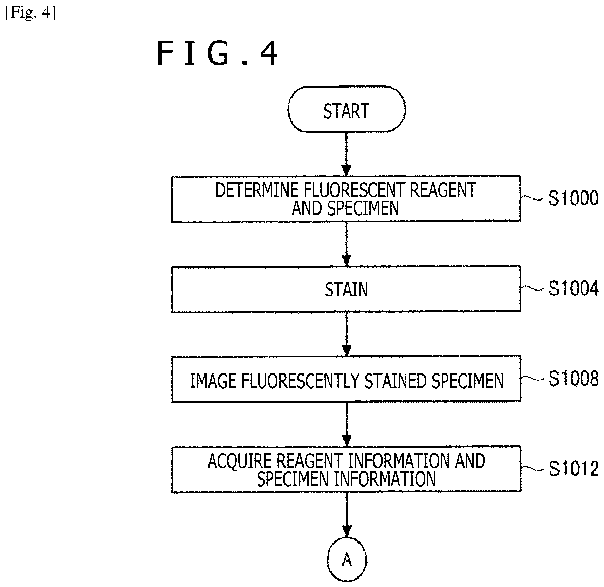

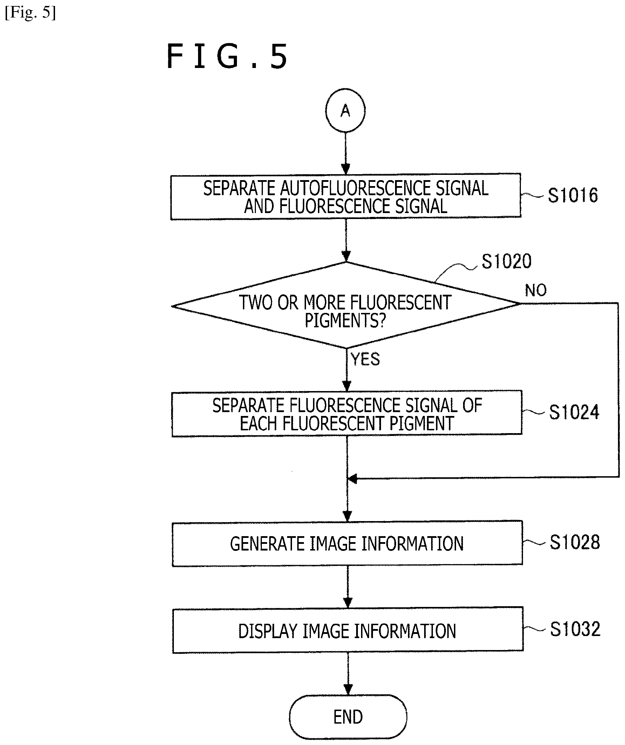

An exemplary configuration of the information processing system according to the present embodiment has been described above. Exemplary flows of processes performed by the information processing apparatus 100 will now be described with reference to Figs. 4 and 5.

In step S1000, the user determines the fluorescent reagent 10 and the specimen 20 that are to be used for analysis. In step S1004, the user prepares the fluorescently stained specimen 30 by staining the specimen 20 with the fluorescent reagent 10.

In step S1008, the image acquisition section 112 of the information processing apparatus 100 acquires the image information by imaging the fluorescently stained specimen 30. In step S1012, the information acquisition section 111 acquires, from the database 200, the reagent information and the specimen information in accordance with the reagent identification information 11 attached to the fluorescent reagent 10 used for the generation of the fluorescently stained specimen 30 and with the specimen identification information 21 attached to the specimen 20.

In step S1016, the analysis section 131 separates the autofluorescence signal of the specimen and the fluorescence signal of the fluorescent reagent 10 from the image information in accordance with the specimen information and the reagent information. Here, in a case where the fluorescence signal includes signals of a plurality of fluorescent pigments ("YES" at step S1020), the analysis section 131 separates the fluorescence signal of each fluorescent pigment in step S1024. It should be noted that in a case where the fluorescence signal does not include signals of a plurality of fluorescent pigments ("NO" at step S1020), the analysis section 131 does not perform the process of separating the fluorescence signal of each fluorescent pigment in step S1024.

In step S1028, the image generation section 132 generates image information by using the fluorescence signal separated by the analysis section 131. For example, the image generation section 132 generates image information by removing the autofluorescence signal from the image information or generates image information indicative of the fluorescence signal of each fluorescent pigment. In step S1032, the display section 140 displays the image information generated by the image generation section 132. This terminates the series of processes.

It should be noted that the steps in the flowcharts of Figs. 4 and 5 need not always be performed chronologically in the depicted order. That is, the steps in the flowcharts may be performed in an order other than depicted or performed in a parallel manner. For example, the analysis section 131 may immediately separate the fluorescence signal of each fluorescent pigment from the image information instead of separating the fluorescence signal of each fluorescent pigment in step S1024 after separating the autofluorescence signal of the specimen and the fluorescence signal of the fluorescent reagent 10 from the image information in step S1016 (further, the analysis section 131 may separate the autofluorescence signal of the specimen from the image information after separating the fluorescence signal of each fluorescent pigment from the image information). Furthermore, the information processing apparatus 100 may additionally perform a process that is not depicted in Fig. 4 or 5. For example, the analysis section 131 may not only separate the signals, but also perform segmentation based on the separated fluorescence signal or autofluorescence signal or analyze the immobilized state of the specimen 20.

<3. Practical Examples>

(3.1. Practical Example of Autofluorescence Signal Removal)

Exemplary flows of various processes performed by the information processing apparatus 100 have been described above. A practical example of autofluorescence signal removal will now be described.

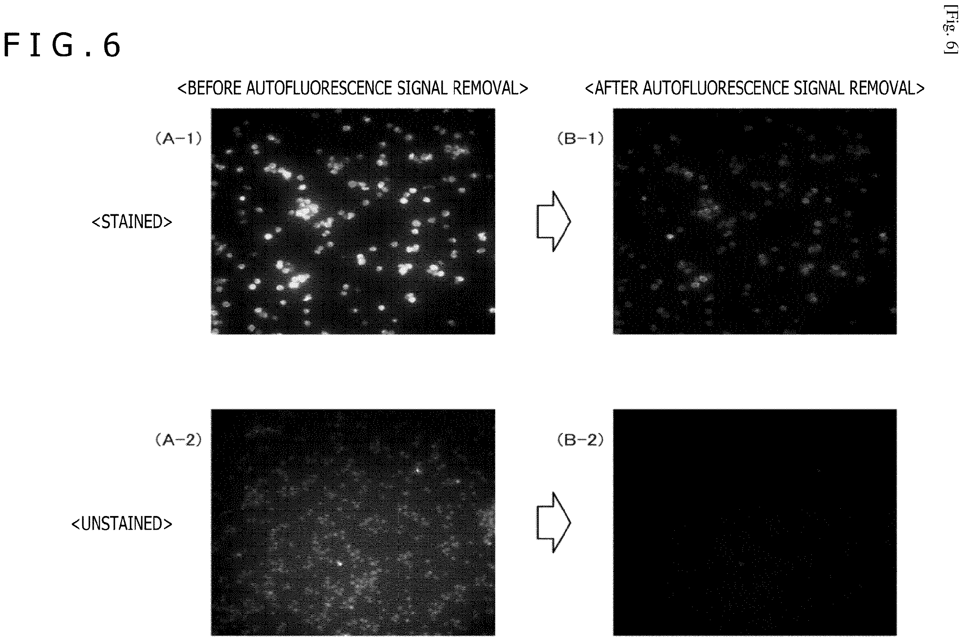

Fig. 6 illustrates an example of image information in a case where K562, which is a chronic myelogenous leukemia cell line, and Jurkat, which is a human T-cell-derived cell line, are used as the specimen 20. More specifically, A-1 of Fig. 6 depicts image information regarding the fluorescently stained specimen 30 that is stained by fluorescent reagents 10, namely, AF488-β-tubulin, AF555-E-cadherin, AF647-CD3, and PE-Cy5-CD45. Further, A-2 of Fig. 6 depicts image information regarding an unstained specimen 20 (image information depicting only the autofluorescence signals of autofluorescent components, namely, NADH, FAD, and Porphin).

Meanwhile, B-1 and B-2 of Fig. 6 respectively depict the image information regarding the fluorescently stained specimen 30 and the image information regarding the specimen 20 that are obtained after allowing the analysis section 131 to separate the fluorescence signal and the autofluorescence signal and removing the autofluorescence signal from the image information. (As mentioned above, the image information depicted in A-2 includes only the autofluorescence signal, and the image information depicted in B-2 includes no signal due to the removal of the autofluorescence signal.)

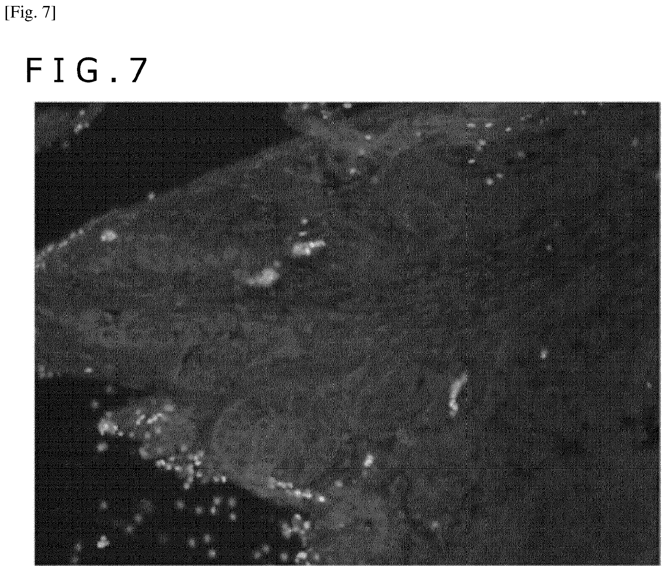

Referring now to Figs. 7 to 9, practical examples of autofluorescence signal removal will be described in relation to a case where a formalin-fixed paraffin-embedded (FFPE: Formalin fixed paraffin embedded) breast cancer slide is used as the specimen 20.

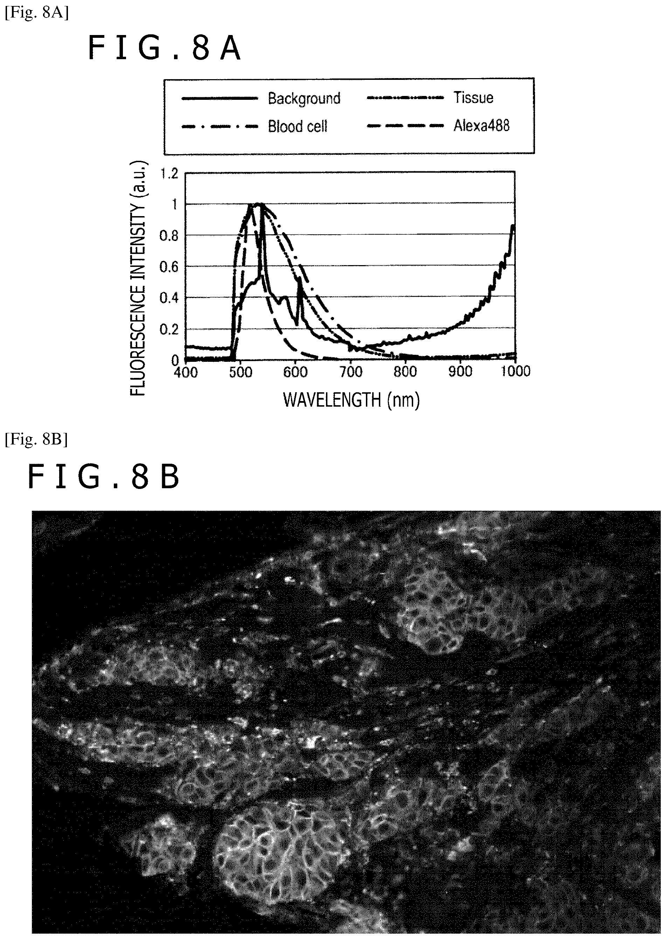

Fig. 7 depicts the image information regarding the fluorescently stained specimen 30 from which the autofluorescence signal is not removed yet. Fig. 8A depicts spectral information regarding the fluorescence signal and the autofluorescence signal. The example of Fig. 8 depicts the spectral information regarding Alexa Fluor 488 (designated as "Alexa 488" in the figure), a background (designated as "Background" in the figure), a tissue (designated as "Tissue" in the figure), and a red blood cell (designated as "Blood cell" in the figure). Fig. 8B depicts, as a comparison target, image information in a case where the signals (the autofluorescence signals of the background, tissue, and red blood cell) of an unstained specimen 20 are measured and then removed from the image information regarding the fluorescently stained specimen 30 (i.e., in a case where the autofluorescence signals are removed by a conventional method).

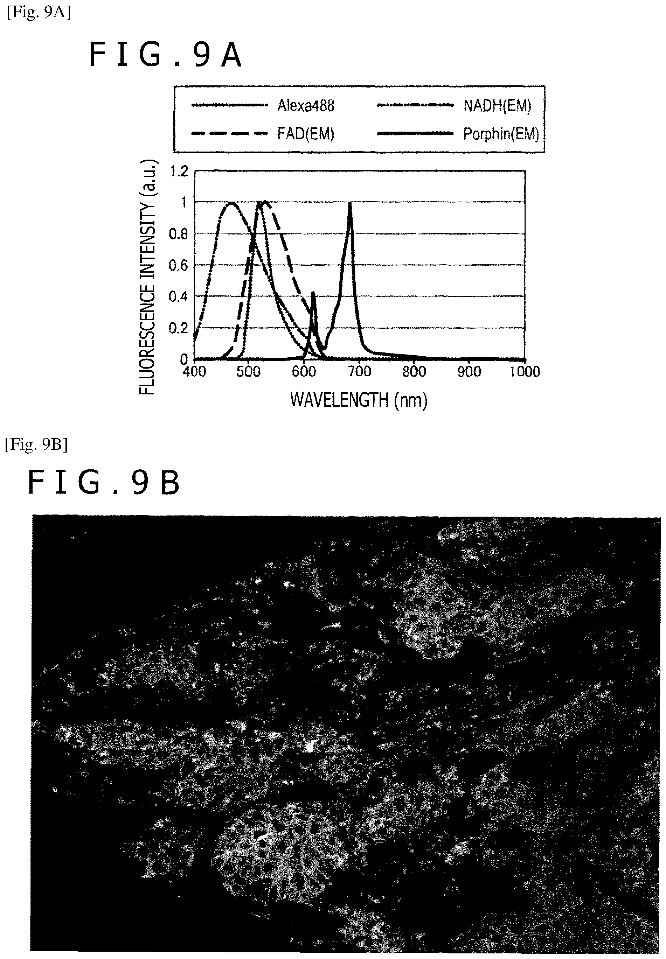

Meanwhile, Fig. 9A depicts the spectral information regarding Alexa Fluor 488 (designated as "Alexa 488" in Fig. 9A), NADH, FAD, and Porphin. Fig. 9B depicts the image information in a case where the analysis section 131 of the information processing apparatus 100 according to the present disclosure removes the autofluorescence signals (the autofluorescence signals regarding NADH, FAD, and Porphin) from the image information regarding the fluorescently stained specimen 30 by using the specimen information and the reagent information.

Obviously, the above description is for exemplification purposes only. The technology according to the present disclosure is applicable to any fluorescent reagent 10 and any specimen 20.

(3.2. Practical Example of Separation of Fluorescence Signals from Each Other)

Further, as described hereinabove, it is possible to confirm specific staining by calculating color separation of an autofluorescence signal and a fluorescence signal from image information obtained by staining specimens 20 using antibody molecules obtained by labeling a plurality of marker molecules individually with a plurality of fluorescence fluorescent dyes and imaging the fluorescently stained specimens 30.

Therefore, in the present practical example, not only separation of an autofluorescence signal and a fluorescence signal and separation of autofluorescence signals from each other but also separation of fluorescence signals from each other are described taking particular examples.

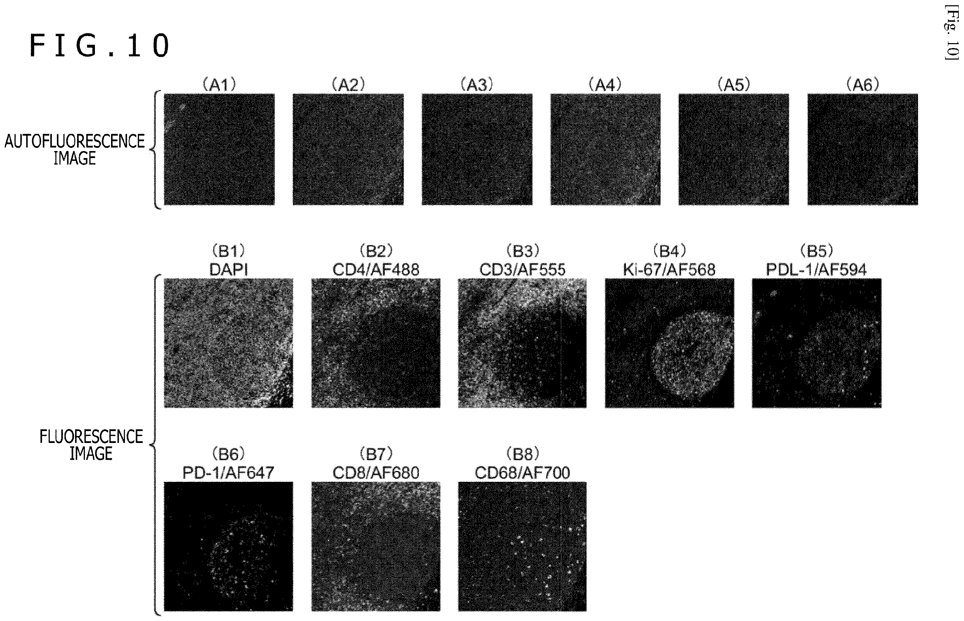

Fig. 10 is a view depicting an example of image information in a case where a tonsil tissue is used as the specimen 20 and an autofluorescence signal obtained from the tonsil tissue is used as the autofluorescence signal to be used for color separation calculation when a fluorescence signal is extracted from image information. In particular, (A1) to (A6) of Fig. 10 depict images of the autofluorescence signal (hereinafter referred to as autofluorescence images) separated from image information, and (B1) to (B8) depict images of the fluorescence signal (hereinafter referred to as fluorescence images) derived from fluorescent reagents 10 separated from the image information.

It is to be noted that, in the example depicted in Fig. 10, a case is indicated in which a human tonsil tissue used as the specimen 20 is stained using eight different fluorescent reagents 10. As the eight different fluorescent reagents 10, in the present example, a fluorescence dye DAPI that is used principally in nuclear staining, a marker antibody CD4 labeled with a fluorescence dye Alexa Fluor 488, a marker antibody CD3 labeled with a fluorescence dye Alexa Fluor 555, a marker antibody Ki-67 labeled with a fluorescence dye Alexa Fluor 568, a marker antibody PDL-1 labeled with a fluorescence dye Alexa Fluor 594, a marker antibody PD-1 labeled with a fluorescence dye Alexa Fluor 647, a marker antibody CD8 labeled with a fluorescence dye Alexa Fluor 680, and a marker antibody CD68 labeled with a fluorescence dye Alexa Fluor 700 are used.

In (A1) to (A6) of Fig. 10, six autofluorescence images derived from a specimen 20 and separated from image information are depicted. Meanwhile, in (B1) of Fig. 10, a fluorescence image derived from the fluorescence dye DAPI and separated from the image information is depicted; in (B2), a fluorescence image derived from the fluorescence dye Alexa Fluor 488 (in Fig. 10, represented as "AF488") and separated similarly from the image information is depicted; in (B3), a fluorescence image derived from the fluorescence dye Alexa Fluor 555 (in Fig. 10, represented as "AF555") and separated similarly from the image information is depicted; in (B4), a fluorescence image derived from the fluorescence dye Alexa Fluor 568 (in Fig. 10, represented as "AF568") and separated similarly from the image information is depicted; in (B5), a fluorescence image derived from the fluorescence dye Alexa Fluor 594 (in Fig. 10, represented as "AF594") and separated similarly from the image information is depicted; in (B6), a fluorescence image derived from the fluorescence dye Alexa Fluor 647 (in Fig. 10, represented as "AF647") and separated similarly from the image information is depicted; in (B7), a fluorescence image derived from the fluorescence dye Alexa Fluor 680 (in Fig. 10, represented as "AF680") and separated similarly from the image information is depicted; and in (B8), a fluorescence image derived from the fluorescence dye Alexa Fluor 700 (in Fig. 10, represented as "AF700") and separated similarly from the image information is depicted.

As can be recognized by referring to (A1) to (A6) and (B1) to (B8) of Fig. 10, according to the present practical example, even where a specimen 20 is stained using a plurality of fluorescent reagents 10, the analysis section 131 can separate an autofluorescence signal and a fluorescence signal from image information obtained from the image acquisition section 112 by imaging a fluorescently stained specimen 30 based on spectrum information of a fluorescence component of the fluorescent reagent 10 and an autofluorescence signal included in reagent information. Then, the analysis section 131 can separate fluorescence signals of the fluorescent reagents 10 from an entire fluorescence signal separated from the autofluorescence signal using spectrum information of the fluorescence components of the fluorescent reagents 10 included in the reagent information. Furthermore, also it is possible for the analysis section 131 to separate individual autofluorescence signals of autofluorescent components from an entire autofluorescence signal after separated from the fluorescence signal using spectrum information of the autofluorescent components included in the specimen information.

In the separation of an autofluorescence signal and a fluorescence signal, for example, a process of fitting an autofluorescence spectrum estimated based on the spectrum information included in the reagent information to the spectrum of the image information and subtracting an autofluorescence signal from the image information based on coefficients in such fitting is executed.

Further, in the separation of autofluorescence signals from each other, a process of fitting the spectra of the fluorescent reagents 10 included in the reagent information to the spectrum of the image sensor after the autofluorescence signal is removed and extracting the fluorescence signal of the fluorescent reagents 10 from the image information based on coefficients upon such fitting is executed.

Furthermore, also in the separation of autofluorescence signals from each other, a process of fitting spectra of the fluorescent reagents 10 included in the reagent information to the spectrum of the image information after the autofluorescence signal is removed and then extracting fluorescence signals of the fluorescent reagents 10 from the image information based on coefficients upon the fitting is executed similarly.

Naturally, the foregoing is exemplary to the last, and the technology according to the present disclosure can be applied to an arbitrary fluorescent reagent 10 and an arbitrary specimen 20.

(3.3. Practical Example of Autofluorescence Signal Used for Extraction of Fluorescence Signal)

Subsequently, a practical example of an autofluorescence signal used for extraction of a fluorescence signal is described. As described hereinabove, in a case where a plurality of specimens 20 exist which are same or similar in the point of view of a tissue used as the specimen 20, a kind of disease of a target, an attribute of a target person, a lifestyle of a target person or the like, the possibility that the autofluorescence signals of the specimens 20 may be similar to each other is high. This is considered similar not only to tissues collected from a same patient but also to tissues collected from different patients.

Therefore, particular examples and advantageous effects achieved by them are described in regard to cases in which, in the present practical example, when a fluorescence signal is extracted from image information, or in other words, when a fluorescence signal and an autofluorescence signal are calculated for color separation, an autofluorescence signal obtained from a tissue same as or similar to the specimen 20 and an autofluorescence signal obtained from a tissue different from the specimen 20 are used. It is to be noted that the color separation process of extracting a fluorescence signal from image information may be, for example, a process corresponding to the "background subtraction process" described hereinabove.

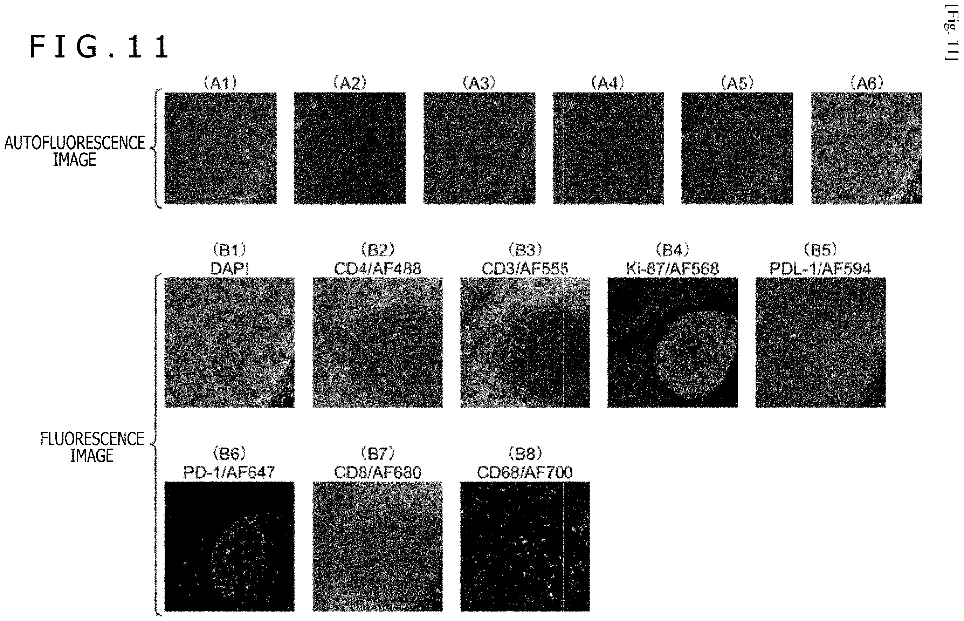

In Fig. 10 referred to in the description of the practical example, a case is exemplified in which a tonsil tissue is used as the specimen 20 and an autofluorescence signal obtained from the tonsil tissue is used as the autofluorescence signal that is used in a color separation process when a fluorescence signal is to be extracted from the image signal. In contrast, Fig. 11 depicts an example of image information in a case where a tonsil tissue is used as the specimen 20 and an autofluorescence signal obtained from a lung tissue, which is a tissue different from the tonsil tissue, is used as the autofluorescence signal that is used for color separation calculation when a fluorescence signal is extracted from image signal.

It is to be noted that, in Fig. 11, six autofluorescence images derived from a specimen 20 and separated from image information are depicted in (A1) to (A6) similarly as in Fig. 10. Further, in (B1) of Fig. 11, a fluorescence image derived from the fluorescence dye DAPI and separated from the image information is depicted; in (B2), a fluorescence image derived from the fluorescence dye Alexa Fluor 488 (in Fig. 11, represented as "AF488") and separated similarly from the image information is depicted; in (B3), a fluorescence image derived from the fluorescence dye Alexa Fluor 555 (in Fig. 11, represented as "AF555") and separated similarly from the image information is depicted; in (B4), a fluorescence image derived from the fluorescence dye Alexa Fluor 568 (in Fig. 11, represented as "AF568") and separated similarly from the image information is depicted; in (B5), a fluorescence image derived from the fluorescence dye Alexa Fluor 594 (in Fig. 11, represented as "AF594") and separated similarly from the image information is depicted; in (B6), a fluorescence image derived from the fluorescence dye Alexa Fluor 647 (in Fig. 11, represented as "AF647") and separated similarly from the image information is depicted; in (B7), a fluorescence image derived from the fluorescence dye Alexa Fluor 680 (in Fig. 11, represented as "AF680") and separated similarly from the image information is depicted; and in (B8), a fluorescence image derived from the fluorescence dye Alexa Fluor 700 (in Fig. 11, represented as "AF700") and separated similarly from the image information is depicted.

As can be recognized from comparison between Fig. 10 and Fig. 11, in a case where an autofluorescence signal derived from a tissue same as or similar to a specimen 20 (in the present example, a tonsil tissue) is used for color separation calculation, a clearer fluorescence image is obtained than in a case where an autofluorescence signal derived from a tissue different from the specimen 20 (in the present example, a lung tissue) is used (refer to (B1) to (B6) of Fig. 10 and (B1) to (B8) of Fig. 11). This indicates that, since an autofluorescence signal on the background is removed more accurately from image information, the S/N ratio of the fluorescence image is improved.

This is described below focusing on a case where the marker antibody CD4 labeled with the fluorescence dye Alexa Fluor 488 is used as the fluorescent reagent 10 ((B1) of Fig. 10 and (B1) of Fig. 11) and another case where the marker antibody PDL-1 labeled with the fluorescence dye Alexa Fluor 594 (refer to (B5) of Fig. 10 and (B5) of Fig. 10) is used as the fluorescent reagent 10.

It is to be noted that, in the present description, the background may be a region that is not stained with the fluorescent reagent 10 or a signal value in the region. Accordingly, the background may include an autofluorescence signal and other noise before a background subtraction process is performed. On the other hand, after the background subtraction process is performed, the background may possibly include an autofluorescence signal, other noise or the like that has not been removed fully.

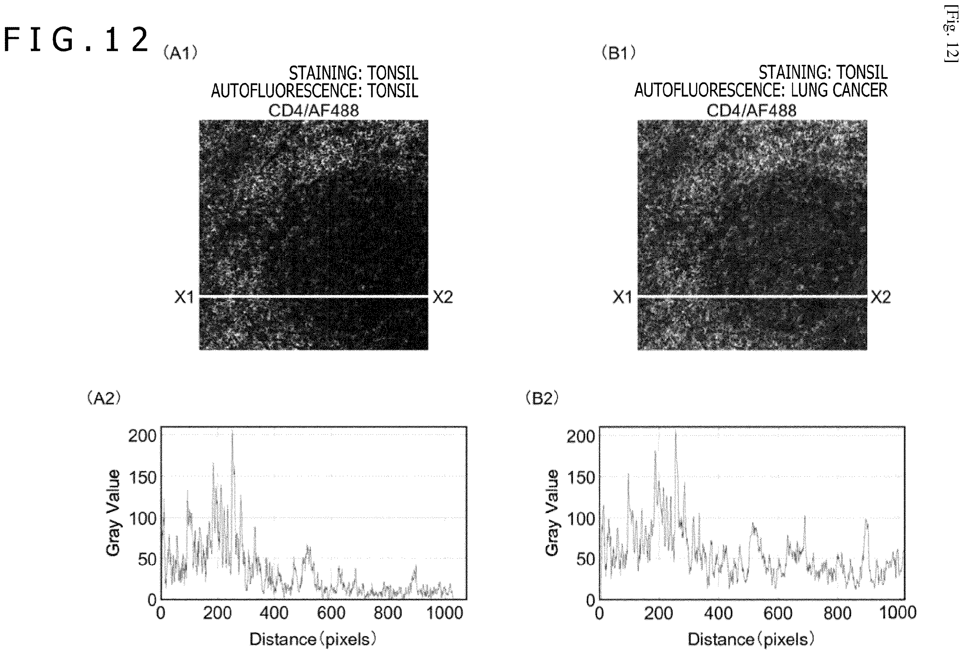

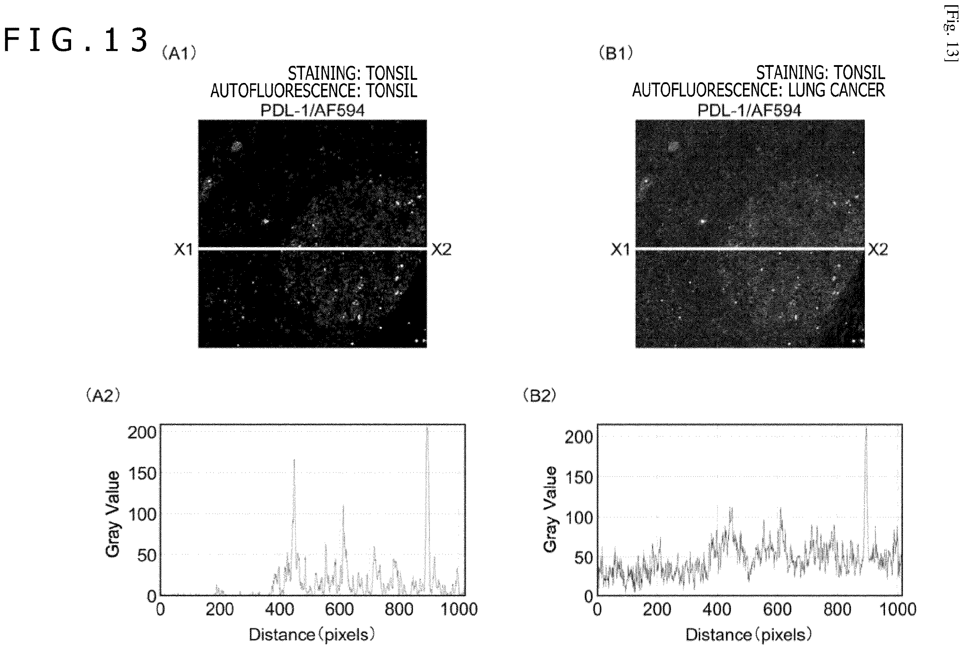

Fig. 12 is a diagram illustrating a case where the marker antibody CD4 labeled with the fluorescence dye Alexa Fluor 488 is used as the fluorescent reagent 10, and Fig. 13 is a diagram illustrating a case where the marker antibody PDL-1 labeled with the fluorescence dye Alexa Fluor 594 is used as the fluorescent reagent 10. Referring to Figs. 12 and 13, (A1) depicts a fluorescence image in a case where an autofluorescence signal derived from a tissue same as or similar to the specimen 20 (in the present example, a tonsil tissue) is used, and (A2) depicts pixel values (Gray Value) along line X1-X2 in (A1). (B1) depicts a fluorescence image in a case where an autofluorescence signal derived from a tissue different from the specimen 20 (in the present example, a lung tissue) is used as the autofluorescence signal to be used for color separation calculation, and (B2) depicts pixel values (Gray Value) along line X1-X2 in (B1). It is to be noted that, in Figs. 12 and 13, X1 corresponds to a pixel (0) at the left end of the fluorescence image, and X2 corresponds to a pixel (1023) at the right end of the fluorescence image.

As apparent from comparison between (A1) and (B1) in Fig. 12 and Fig. 13, in a case where an autofluorescence signal derived from a tissue same as or similar to a specimen 20 (in the present example, a tonsil tissue) is used as an autofluorescence signal to be used for color separation calculation, the signal value (corresponding to a pixel value) on the background is reduced from that of the image information by a greater amount than in a case where an autofluorescence signal derived from a tissue different from the specimen 20 (in the present example, a lung tissue) is used. As a result, the S/N ratio in the fluorescence image is reduced and the fluorescence image obtained is clearer.

It is considered that this arises from the fact that the autofluorescence spectrum derived from a same or similar tissue is nearer to the autofluorescence signal of the specimen 20 than the autofluorescence signal derived from a different tissue.

From this, it can be considered that it is preferable to use, as the autofluorescence signal to be used for color separation calculation, the autofluorescence signal derived from a tissue same as or similar to the specimen 20. However, this does not exclude it from the technical scope of the present disclosure that an autofluorescence signal derived from a tissue different from the specimen 20 is used for color separation calculation.

Meanwhile, information regarding an autofluorescence signal derived from a tissue same as or similar to the specimen 20 may be managed, for example, by the database 200. The information acquisition section 111 can acquire specimen information of a specimen same as or similar to the specimen 20 from the database 200 based on the acquired specimen identification information 21 and input the acquired specimen information to the analysis section 131 through the information storage section 121. The analysis section 131 is based on the inputted specimen information to execute such processes as separation of an autofluorescence signal and a fluorescence signal, separation of fluorescence signals from each other and separation of autofluorescence signals from each other.

(3-4. Analysis of Immobilized State)

A practical example of autofluorescence signal removal has been described above. A practical example of analysis of the immobilized state of the specimen 20 will now be described.

As described earlier, the overall autofluorescence signal of the specimen 20 varies with the immobilized state of the specimen 20 (e.g., immobilization method). Therefore, the information processing apparatus 100 is able to analyze (evaluate) the immobilized state of the specimen 20 in accordance with the autofluorescence signal component ratio of each of two or more autofluorescence-emitting components. More specifically, as far as NADPH (nicotinamide adenine dinucleotide phosphate reduced form), FAD, and other autofluorescent components are concerned, the fluorescence intensity varies without substantially changing the shape of the autofluorescence spectrum depending on immobilization by formalin. Consequently, as regards the above-mentioned autofluorescent components, the information processing apparatus 100 is able to analyze the immobilized state of the specimen 20 (e.g., immobilization method) in accordance with the fluorescence intensity ratio of each autofluorescent component separated from the image information.

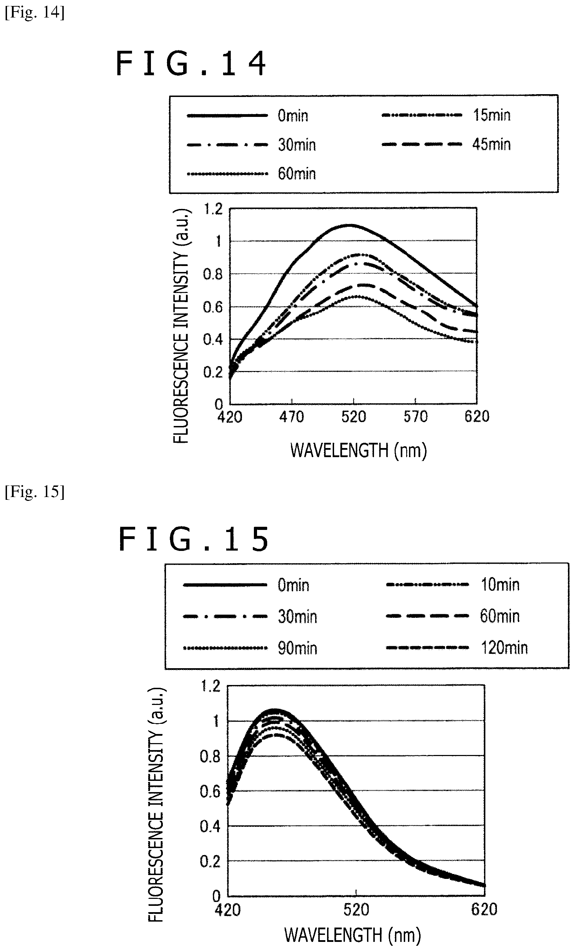

Referring now to Figs. 14 to 17, the following describes changes in the autofluorescence spectrum in a case where various autofluorescent components are immobilized by formalin.

Fig. 14 is a diagram illustrating autofluorescence spectra measured at the beginning of immobilization (zero minutes) and at 15, 30, 45, and 60 minutes after immobilization in a case where K562, which is a chronic myelogenous leukemia cell line, is immobilized by formalin having a concentration of 5%. As far as K562 is concerned, Fig. 14 indicates that as the period of time during which immobilization is provided by formalin becomes longer, the shape of the autofluorescence spectra changes to decrease the fluorescence intensity. It should be noted that "the shape of the autofluorescence spectra changes" indicates that the autofluorescence spectra do not overlap with each other even when the autofluorescence spectra acquired at each point of time are moved in parallel.

Fig. 15 is a diagram illustrating autofluorescence spectra measured at the beginning of immobilization (zero minutes) and at 10, 30, 60, 90, and 120 minutes after immobilization in a case where NADPH is immobilized by formalin having a concentration of 2%. As far as NADPH is concerned, Fig. 15 indicates, in marked contrast to the above-described K562, that the fluorescence intensity decreases without substantially changing the shape of the autofluorescence spectra even when the period of time during which immobilization is provided by formalin becomes longer. It should be noted that "the fluorescence intensity decreases without substantially changing the shape of the autofluorescence spectra" indicates that the autofluorescence spectra substantially overlap with each other when the fluorescence intensity of the autofluorescence spectra acquired at each point of time is vertically varied (i.e., when the autofluorescence spectra are vertically moved in parallel).

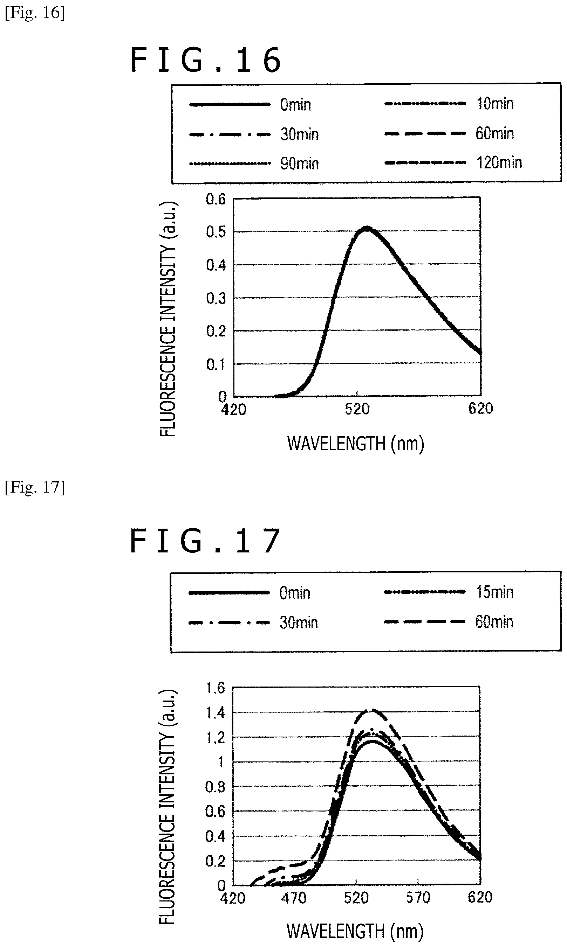

Fig. 16 is a diagram illustrating autofluorescence spectra measured at the beginning of immobilization (zero minutes) and at 10, 30, 60, 90, and 120 minutes after immobilization in a case where FAD (30 $\mu$M) is immobilized by formalin having a concentration of 2%. As far as FAD (30 $\mu$M) is concerned, Fig. 16 indicates that the shape and fluorescence intensity of the autofluorescence spectra do not substantially change even when the period of time during which immobilization is provided by formalin becomes longer.

Fig. 17 is a diagram illustrating autofluorescence spectra measured at the beginning of immobilization (zero minutes) and at 15, 30, and 60 minutes after immobilization in a case where FAD (50 $\mu$M) is mixed with cell extract and immobilized by formalin having a concentration of 2%. As far as FAD (50 $\mu$M) is concerned, Fig. 17 indicates that the fluorescence intensity increases without substantially changing the shape of the autofluorescence spectra even when the period of time during which immobilization is provided by formalin becomes longer.

As indicated by the examples of Figs. 15 to 17, as far as NADPH, FAD, and other autofluorescent components are concerned, the fluorescence intensity varies without substantially changing the shape of an autofluorescence spectrum depending on the immobilized state (e.g., immobilization method) (it should be noted that, in some cases, the fluorescence intensity may also remain substantially unchanged as indicated in Fig. 16). Here, when the specimen information acquired from the database 200 by the information acquisition section 111 includes spectral information depicted in Figs. 15 to 17, the analysis section 131 is able to analyze (evaluate) the immobilized state of the specimen 20 in accordance with the autofluorescence signal component ratio of each of two or more autofluorescence-emitting components.

For example, the analysis section 131 recognizes the autofluorescent components included in the specimen 20 in accordance with the measurement channel included in the specimen information, and acquires, as the specimen information, the spectral information regarding each autofluorescent component, which varies depending on immobilization. The analysis section 131 is then able to analyze the immobilization method (e.g., a chemical used for immobilization, the concentration of the chemical, or the process of immobilization) and the elapsed time since immobilization in accordance with the acquired spectral information regarding each autofluorescent component and with the component ratio of each component of the autofluorescence signal separated from the image information.

Although the foregoing description assumes that NADPH and FAD are the autofluorescent components, the type of autofluorescent component applicable to the analysis of the immobilized state is not limited to the above ones. That is, autofluorescent components whose autofluorescence spectrum remains substantially unchanged in shape as a result of immobilization are applicable to the analysis of the immobilized state.

(3-5. Segmentation)

A practical example of analysis of the immobilized state of the specimen 20 has been described above. The following describes segmentation (or region segmentation) for recognizing a region of an object (e.g., cell, intracellular structure (cytoplasm, cell membrane, or nucleus), or tissue (tumor part, non-tumor part, connective tissue, blood vessel, vascular wall, lymphatic vessel, fibrosis structure, or necrosis)) included in the image information.

In order to acquire the fluorescence signal to be measured, the method of amplifying the fluorescence signal until the autofluorescence signal derived from the specimen 20 is negligible has been conventionally and widely used. However, when this method is used, it is difficult to acquire the autofluorescence signal. Therefore, when the conventional method is used, it is difficult to recognize, by using the autofluorescence signal, a region of a cell, intracellular structure (cytoplasm, cell membrane, nucleus, etc.), or tissue (tumor part, non-tumor part, connective tissue, blood vessel, vascular wall, lymphatic vessel, fibrosis structure, necrosis, etc.) included in the specimen 20.

Meanwhile, the analysis section 131 of the information processing apparatus 100 is able to extract the autofluorescence signal from the image information regarding the fluorescently stained specimen 30 in accordance with the specimen information and the reagent information. Therefore, in accordance with the distribution of the autofluorescence signal and fluorescence signal in the image information, the analysis section 131 is able to recognize a region of a cell, intracellular structure (cytoplasm, cell membrane, nucleus, etc.), or tissue (tumor part, non-tumor part, connective tissue, blood vessel, vascular wall, lymphatic vessel, fibrosis structure, necrosis, etc.) included in the specimen 20, and perform segmentation. This enables the analysis section 131 to acquire localization information regarding a target molecule and achieve, for example, image information positioning between different stains.

In the past, there has been a proposed method for performing segmentation by attaching a unique label to each cell and each intracellular structure. There has been another proposed method for correcting the expression level of a target molecule in a cell membrane by labeling, with different fluorescence, a molecule (endogenous control) that is different from the target molecule and regularly expressed in the same region as for the target molecule. The autofluorescent components have a unique localization. Therefore, using the localization information regarding the autofluorescent components, which is acquired in accordance with the present disclosure, makes it possible to improve the accuracy of processing while reducing the number of stains for conventional segmentation and endogenous control. It should be noted that the method of segmentation based on the technology according to the present disclosure is not limited to the above-described one.

<4. Exemplary Hardware Configuration>

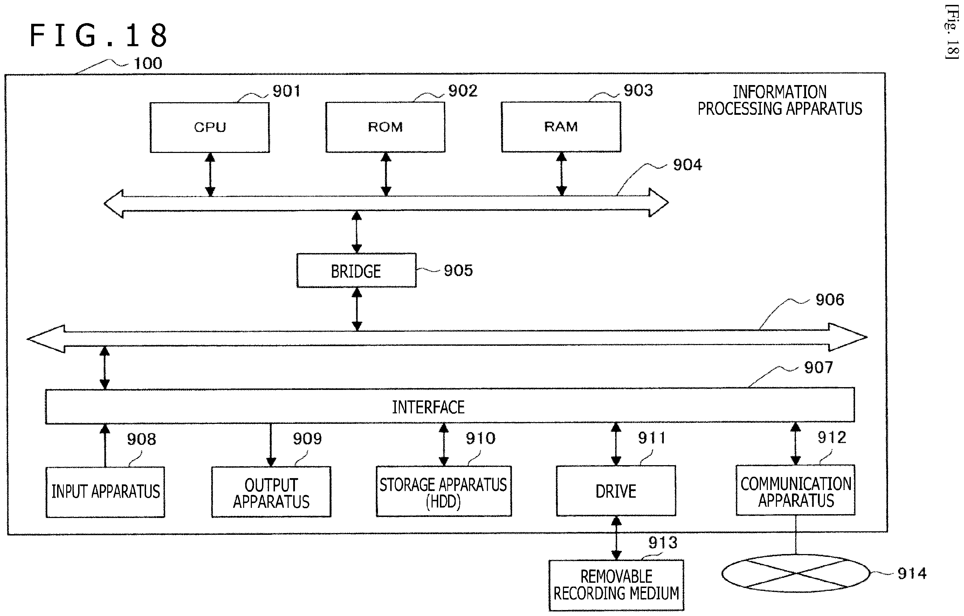

Segmentation based on the technology according to the present disclosure has been described above. An exemplary hardware configuration of the information processing apparatus 100 will now be described with reference to Fig. 18.

Fig. 18 is a block diagram illustrating an exemplary hardware configuration of the information processing apparatus 100. The information processing apparatus 100 includes a CPU (Central Processing Unit) 901, a ROM (Read Only memory) 902, a RAM (Random Access Memory) 903, a host bus 904, a bridge 905, an external bus 906, an interface 907, an input apparatus 908, an output apparatus 909, a storage apparatus (HDD) 910, a drive 911, and a communication apparatus 912.

The CPU 901 functions as an arithmetic processing apparatus and a control apparatus, and provides overall control of operations in the information processing apparatus 100 in accordance with various programs. Further, the CPU 901 may be a microprocessor. The ROM 902 stores, for example, programs and operating parameters used by the CPU 901. The RAM 903 temporarily stores, for example, the programs used in an execution by the CPU 901 and parameters changing as appropriate in such an execution. These components are interconnected by the host bus 904, which includes, for example, a CPU bus. The CPU 901, the ROM 902, and the RAM 903 cooperate to implement the functions of the processing section 130 and control section 150.

A controlling program for an image processing apparatus according to the present technology is a program for causing the information processing apparatus to execute a function similar to the control function performed by the control section 150 of the information processing apparatus described above and is provided in such a state that it is stored in a recording medium such as, for example, a magnetic disk, an optical disk, a magneto-optical disk or a flash memory such that it is loaded into and used by a PC or the like. As an alternative, also it is possible to download and use the controlling program delivered from the outside through a network such as the Internet into and with a PC or the like.

In particular, also it is possible to consider that the information processing system according to the present embodiment (for example, a microscope system) is configured from the information processing apparatus 100 described hereinabove and a program for causing the information processing apparatus 100 to execute a control function for controlling so as to separate a fluorescence signal and/or an autofluorescence signal from image information based on information regarding the specimen 20 and information regarding the fluorescent reagent 10.

The host bus 904 is connected to the external bus 906, such as a PCI (Peripheral Component Interconnect/Interface) bus, through the bridge 905. It should be noted that the host bus 904, the bridge 905, and the external bus 906 need not always be provided separately. Alternatively, a single bus may be adopted to implement the functions of the host bus 904, the bridge 905, and the external bus 906.

The input apparatus 908 includes, for example, an input control circuit that functions as a user's information input apparatus, such as a mouse, a keyboard, a touch panel, a button, a microphone, a switch, a lever, or various sensors (including an imaging element), generates an input signal based on a user input, and outputs the generated input signal to the CPU 901. The input apparatus 908 implements some of the functions of the acquisition section 110 and the functions of the operation section 160.

The output apparatus 909 includes, for example, a display apparatus such as a CRT (Cathode Ray Tube) display apparatus, a liquid-crystal display (LCD) apparatus, an OLED (Organic Light Emitting Diode) apparatus, or a lamp. Further, the output apparatus 909 includes an audio output apparatus such as speakers or headphones. The output apparatus 909 displays various information, such as video data, as images or text. Meanwhile, the audio output apparatus converts, for example, audio data to a sound and outputs the sound. The output apparatus 909 implements the functions of the display section 140.

The storage apparatus 910 is an apparatus for data storage. The storage apparatus 910 may include, for example, a storage medium, a recording apparatus for recording data on the storage medium, a reading apparatus for reading the data from the storage medium, and a deletion apparatus for deleting the data recorded on the storage medium. For example, an HDD (Hard Disk Drive) may be used as the storage apparatus 910. In this case, the storage apparatus 910 drives a hard disk so as to store a program to be executed by the CPU 901 and various data. The storage apparatus 910 implements the functions of the storage section 120.

The drive 911 is a reader/writer for a storage medium, and built in or externally attached to the information processing apparatus 100. The drive 911 reads information recorded on a removable storage medium 913, such as an inserted magnetic disk, optical disk, a magneto-optical disk, or a semiconductor memory, and outputs the read information to the RAM 903. Further, the drive 911 is able to write information onto the removable storage medium 913.

The communication apparatus 912 is, for example, a communication interface that includes a communication device for connecting to a communication network 914. The communication apparatus 912 implements some of the functions of the acquisition section 110.

It should be noted that the hardware configuration of the information processing apparatus 100 is not limited to that depicted in Fig. 18. For example, in a case where communication is to be established through a connected external communication device, the information processing apparatus 100 need not include the communication apparatus 912. Further, the communication apparatus 912 may be capable of establishing communication by a plurality of communication methods. Furthermore, the configuration depicted in Fig. 18 may be wholly or partially implemented by one or two or more ICs (Integrated Circuits).

<5. Remarks>

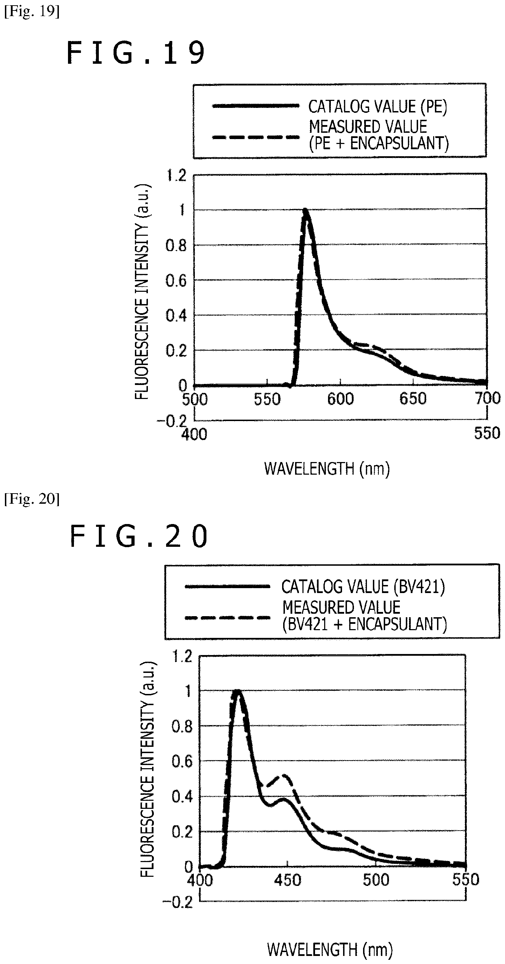

The foregoing description indicates that the specimen information and the reagent information are preferably measured and managed in a unique manner within the information processing system according to the present disclosure. The reason is that actual specimen information and reagent information differ from catalog values and literature values in many cases. Therefore, referring now to Figs. 19 and 20, the difference of the actual specimen information and reagent information from the catalog values and literature values will be described as remarks.

Fig. 19 is a diagram illustrating the result of comparison between measured values and catalog values indicative of the spectral information regarding PE (Phycoerythrin), which is a kind of fluorescent component. Further, Fig. 20 is a diagram illustrating the result of comparison between measured values and catalog values indicative of the spectral information regarding BV421 (Brilliant Violet 421), which is a kind of fluorescent component. It should be noted that the measured values indicative of measurement results are obtained when samples are prepared by mixing the fluorescent components with an encapsulant.

As illustrated in Figs. 19 and 20, the measured values and catalog values substantially agree with each other in the peak position indicated by the spectral information, but differ in the spectral shape at wavelengths longer than the peak wavelength. Therefore, using the catalog values as the spectral information decreases the accuracy of separation of the fluorescence signal and autofluorescence signal.

From the viewpoint of accuracy, it should be noted in general that not only the spectral information but also various information included in the specimen information and reagent information is preferably measured in a unique manner within the information processing system according to the present disclosure.

<6. Modification of System Configuration>

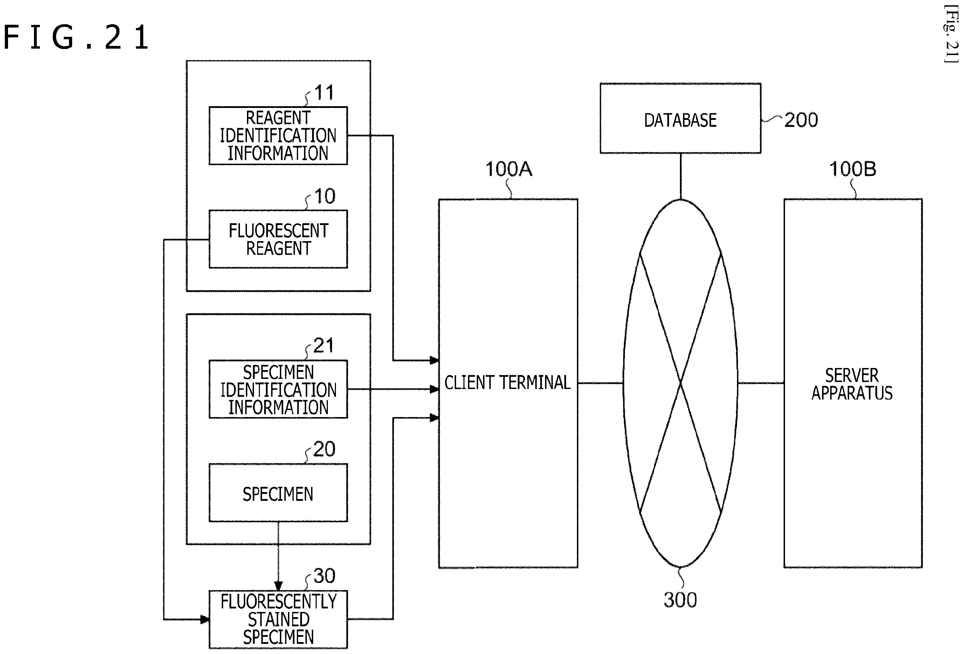

It is to be noted that also it is possible for the information processing system according to the embodiment described above (refer to Fig. 3) to have a server-client type system configuration. Fig. 21 is a block diagram depicting a schematic configuration example of an information processing system configured in a server-client type configuration.

As depicted in Fig. 21, the information processing system according to the present modification includes a client terminal 100A, a server apparatus 100B and a database 200, which are connected for communication with each other through a predetermined network 300. To the network 300, various networks such as a WAN (Wide Area Network) (including the Internet), a LAN (Local Area Network), a public network and a mobile network can be applied.

The client terminal 100A is a terminal apparatus used by a doctor, a researcher or the like and includes at least, for example, the acquisition section 110, display section 140, control section 150 and operation section 160 in the configuration depicted in Fig. 3.

On the other hand, the server apparatus 100B is not limited to a single server and may be configured from a plurality of servers or may be a cloud server. The server apparatus 100B can include at least one of the information storage section 121, image information storage section 122, analysis result storage section 123, analysis section 131, or image generation section 132 in the configuration depicted in Fig. 3. Any component that is not included in the server apparatus 100B from among the components mentioned may be provided in the client terminal 100A.

The number of such client terminals 100A connected to the server apparatus 100B is not limited to one but may be a plural number. In this case, the plurality of client terminals 100A may be introduced in hospitals different from each other.

By adopting such a system configuration as described above, it is possible not only to provide a system having a higher calculation capacity to a user such as a doctor or a researcher but also to accumulate a greater amount of information into the database 200. This suggests facilitating extension to a system that executes machine learning or the like for big data accumulated in the database 200.

It is to be noted that such a configuration as described above is not restrictive and various alterations are possible, for example, like a configuration that only the database 200 is shared by a plurality of information processing apparatus 100 through the network 300.

<7. Conclusions>

As described above, based on the specimen information and the reagent information, the information processing apparatus 100 according to the present disclosure is able to separate the autofluorescence signal of the specimen 20 and the fluorescence signal of the fluorescent reagent 10 from the image information regarding the specimen 20 stained by at least one fluorescent reagent 10. Further, the information processing apparatus 100 is able to perform various processes (e.g., the analysis of the immobilized state of the specimen 20 or the segmentation) by using the separated fluorescence signal or autofluorescence signal. Moreover, the information processing apparatus 100 is able to improve the above-described processes, such as the process of separating the autofluorescence signal and the fluorescence signal, by performing machine learning based on the specimen information, reagent information, and analysis results managed by the database 200.

<8. Exemplary Application>

The technology according to the present disclosure can be applied to various produces. For example, the technology according to the present disclosure may be applied to a pathological diagnosis system for allowing a doctor or the like to observe a cell or a tissue collected from a patient to diagnose a lesion or a support system for the same (hereinafter referred to as diagnosis supporting system). This diagnosis supporting system may be a DPI (Digital Pathology Imaging) system for diagnosing a lesion based on an image acquired utilizing a digital pathology technology or for supporting the diagnosis.

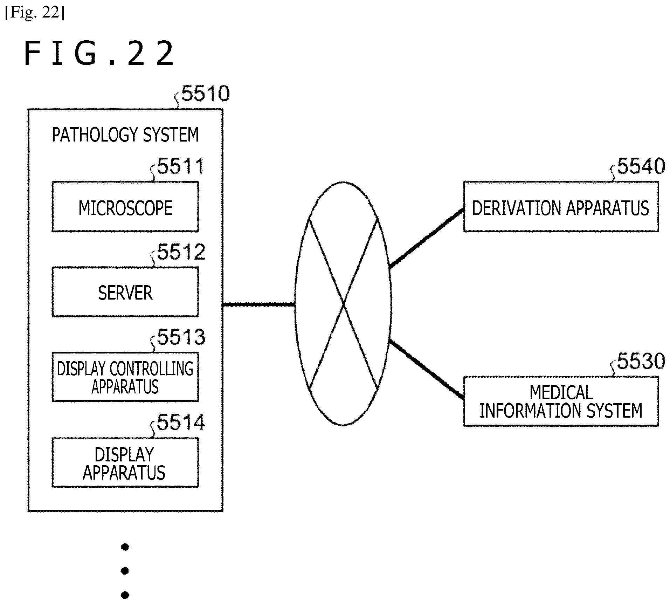

Fig. 22 is a diagram depicting an example of a schematic configuration of a diagnosis supporting system 5500 to which the technology according to the present disclosure is applied. As depicted in Fig. 22, the diagnosis supporting system 5500 includes one or more pathology systems 5510, a medical information system 5530, and a derivation apparatus 5540.

Each of the one or more pathology systems 5510 is a system used principally by a pathologist and is introduced, for example, into a laboratory or a hospital. The pathology systems 5510 may be introduced into hospitals different from each other and are individually connected to the medical information system 5530 and the derivation apparatus 5540 through various networks such as a WAN (Wide Area Network) (including the Internet), a LAN (Local Area Network), a public network, or a mobile network.

Each pathology system 5510 includes a microscope 5511, a server 5512, a display controlling apparatus 5513, and a display apparatus 5514.

The microscope 5511 has a function of an optical microscope, and images an observation target fitted in a glass slide to acquire a pathological image in the form of a digital image. The observation target is, for example, a tissue or a cell collected from a patient and may be a piece of meat of an organ, saliva, blood or the like.

The server 5512 stores and retains a pathological image acquired by the microscope 5511 into a storage section not depicted. Further, in a case where the server 5512 accepts a reading request from the display controlling apparatus 5513, it searches for a pathological image from the storage section not depicted and sends the searched out pathological image to the display controlling apparatus 5513.

The display controlling apparatus 5513 sends a reading request for a pathological image accepted from the user to the server 5512. Then, the display controlling apparatus 5513 controls the display apparatus 5514, which uses a liquid crystal unit, an EL (Electro-Luminescence) unit, a CRT (Cathode Ray Tube) or the like, to display the pathological image accepted from the server 5512. It is to be noted that the display apparatus 5514 may be ready for a 4K or 8K display system, and the number of such display apparatus 5514 is not limited to one but may be a plural number.

Here, in a case where the observation target is a solid such as a piece of meat of an organ, the observation target may be, for example, a stained thin slice. The thin slice may be produced by thinly cutting a block piece cut out from a sample of an organ or the like. Further, upon thin cutting, the block piece may be fixed by paraffin or the like.

For staining of a thin slice, various staining methods may be applied such as general staining that indicates the form of an organ such as HE (Hematoxylin-Eosin) staining or immunostaining that indicates an immune status of an organ such as IHC (Immunohistochemistry) staining. Along with this, one thin slice may be stained using a plurality of different reagents, or two or more thin slices cut out successively from a same block piece (also referred to as adjacent thin slices) may be stained using reagents different from each other.

The microscope 5511 can include a low resolution imaging section for imaging with a low resolution and a high resolution imaging section for imaging with a high resolution. The low resolution imaging section and the high resolution imaging section may be optical systems different from each other or may be a same optical system. In a case where they are a same optical system, the microscope 5511 may have a resolution that changes in response to an imaging target.

A glass slide in which an observation target is fitted is placed on a stage positioned within an angle of view of the microscope 5511. The microscope 5511 first acquires a whole image in the angle of view using the low resolution imaging section and specifies a region of an observation target from the acquired whole image. Then, the microscope 5511 divides the region in which the observation target exists into a plurality of divisional regions of a predetermined size and successively images the divisional regions with the high resolution imaging section to acquire high resolution images of the individual divisional regions. Upon switching of the target divisional region, the stage may be moved or the imaging optical system may be moved, or else both of them may be moved. Further, each divisional region may overlap with an adjacent divisional region in order to prevent occurrence of an imaging leakage region by unintended displacement of the glass slide or from a like reason. Furthermore, the whole image may include identification information for associating the whole image and the patient with each other. This identification information may be, for example, a character string, a QR code (registered trademark) or the like.

The medical information system 5530 is a so-called electronic medical record system and stores information for identifying a patient, disease information of the patient, inspection information and image information used in diagnosis, and information regarding diagnosis such as a diagnosis result, prescription drugs and so forth. For example, a pathological image obtained by imaging an observation target of a certain patient is retained once through the server 5512 and can be displayed on the display apparatus 5514 by the display controlling apparatus 5513. A pathologist who utilizes the pathology system 5510 would perform pathological diagnosis based on the pathological image displayed on the display apparatus 5514. A result of the pathological diagnosis conducted by the pathologist is stored into the medical information system 5530.

The derivation apparatus 5540 can execute analysis for a pathological image. For this analysis, a learning model generated by mechanical learning can be used. The derivation apparatus 5540 may derive a classification result of a specific region, an identification result of a tissue and so forth. Further, the derivation apparatus 5540 may derive identification results of cell information, a quantity, a position, luminance information and so forth, scoring information regarding them and so forth. The information derived by the derivation apparatus 5540 may be displayed as diagnosis supporting information on the display apparatus 5514 of the pathology system 5510.

The technology according to the present disclosure can be applied suitably to the overall diagnosis supporting system 5500 in the constitution elements described hereinabove. In particular, the acquisition section 110 corresponds to the microscope 5511; the display controlling apparatus 5513 corresponds to the control section 150; the display apparatus 5514 corresponds to the display section 140; the remaining components of the pathology system 5510 and the derivation apparatus 5540 correspond to the remaining components of the information processing apparatus 100; and the medical information system 5530 can correspond to the database 200. By applying the technology according to the present disclosure to the diagnosis supporting system 5500, such an advantageous effect can be demonstrated that it becomes possible to allow a doctor or a researcher to perform diagnosis or analysis of a lesion more accurately.

While the preferred embodiment according to the present disclosure has been described in detail with reference to the accompanying drawings, the technical scope of the present disclosure is not limited to the above-described one. It is obvious to those having ordinary skill in the particular art of the present disclosure that various changes and modifications may be made without departing from the scope of the present disclosure as defined in the following claims. It is to be understood that such changes and modifications are within the technical scope of the present disclosure.

Further, the advantages described in the present specification are explanatory or illustrative and not restrictive. That is, in addition to or in place of the above-described advantageous effects, the technology according to the present disclosure may provide other advantages that may be contemplated by those skilled in the art, provided that they are within the scope of the appended claims, upon consideration of the description in the present specification.

[Reference Signs List]

10 Fluorescent reagent
11 Reagent identification information
20 Specimen
21 Specimen identification information
30 Fluorescently stained specimen
100 Information processing apparatus
100A Client terminal
100B Server apparatus
110 Acquisition section
111 Information acquisition section
112 Image acquisition section
120 Storage section
121 Information storage section
122 Image information storage section

123 Analysis result storage section
130 Processing section
131 Analysis section
132 Image generation section
140 Display section
150 Control section
160 Operation section
200 Database
300 Network

**Claims**

1. An information processing apparatus (100), comprising:

   an image acquisition section that acquires image information of a specimen (20) stained by a fluorescence reagent (10);
   an information acquisition section (112) that acquires measurement channel indicative of a first autofluorescent component and a second autofluorescent component included in the specimen and spectral information regarding an autofluorescent spectrum possessed by each of the first autofluorescent component and the second autofluorescent component included in the specimen and spectral information regarding a fluorescence spectrum possessed by fluorescent components included in the fluorescence reagent; and
   an analysis section (131) that

   separates a first fluorescence signal from the image information in accordance with the measurement channel and spectral information regarding the specimen and separates a second fluorescence signal from the image information in accordance with the spectral information regarding the fluorescent components included in the fluorescence reagent,
   wherein the first fluorescence signal includes an autofluorescence signal of the first autofluorescent component and an autofluorescence signal of the second autofluorescent component, wherein the spectral information of each of the autofluorescent components varies depending on immobilization and wherein the second fluorescence signal includes a fluorescence signal of the fluorescence reagent,
   performs segmentation, based on the separated first and second fluorescence signals, for recognising a region of an object or tissue included in the image information, and
   analyzes a fixation state of the specimen in accordance with a component ratio of each of the autofluorescence signal of the first autofluorescent component and the autofluorescence signal of the second autofluorescent component.

2. The information processing apparatus according to claim 1, wherein the autofluorescent component included in the specimen includes at least one or more of NADH, i.e. reduced nicotinamide adenine dinucleotide, FAD, i.e. flavin adenine dinucleotide, or Porphin.

3. The information processing apparatus according to claim 1, wherein
   the information regarding the specimen further includes information regarding a kind of tissue to be used, a kind of disease of a target, an attribute of a target person, and a lifestyle habit of the target person.

4. The information processing apparatus according to claim 1, wherein
   the information acquisition section acquires the information regarding the specimen in accordance with specimen identification information capable of identifying the specimen and acquires the information regarding the fluorescence reagent in accordance with reagent identification information capable of identifying the fluorescence reagent.

5. The information processing apparatus according to claim 4, wherein
   the reagent identification information is further capably of identifying a production lot of the fluorescence reagent.

6. The information processing apparatus according to claim 1, further comprising:
   a display section (140) that displays image information generated based on the first fluorescence signal or the second fluorescence signal.

7. The information processing apparatus according to claim 1, wherein

the information regarding the fluorescence reagent includes a quantum yield, a fluorescent labeling ratio, and an absorption cross section or a molar absorption coefficient concerning the fluorescent reagent; and
the analysis section calculates at least one of the number of fluorescence molecules in the image information or the number of antibodies coupled to the fluorescence molecules using the image information from which the autofluorescence signals are
removed and information regarding the fluorescence reagent.

8. A microscope system, comprising:

an information processing apparatus (100) according to claim 1; and
a program for causing the information processing apparatus to execute a controlling function for controlling so as:
to separate said first fluorescence signal and to separate said second fluorescence to perform said segmentation, and to analyze said fixation state of the specimen.

**Patentansprüche**

1. Informationsverarbeitungseinrichtung (100), umfassend:

einen Bilderfassungsabschnitt, der Bildinformationen einer Probe (20) erfasst, die durch ein Fluoreszenzreagenz (10) gefärbt wird;
einen Informationserfassungsabschnitt (112), der Messkanäle, die eine erste Autofluoreszenzkomponente und eine zweite Autofluoreszenzkomponente anzeigen, die in der Probe eingeschlossen sind, und Spektralinformationen bezüglich eines Autofluoreszenzspektrums, das jede der ersten Autofluoreszenzkomponente und der zweiten Autofluoreszenzkomponente aufweist, die in der Probe eingeschlossen sind, und Spektralinformationen bezüglich eines Fluoreszenzspektrums erfasst, das Fluoreszenzkomponenten aufweist, die in dem Fluoreszenzreagenz eingeschlossen sind; und
einen Analyseabschnitt (131), der
ein erstes Fluoreszenzsignal aus den Bildinformationen gemäß dem Messkanal und Spektralinformationen bezüglich der Probe trennt und ein zweites Fluoreszenzsignal aus den Bildinformationen gemäß den Spektralinformationen bezüglich der Fluoreszenzkomponenten trennt, die in dem Fluoreszenzreagenz eingeschlossen sind, wobei das erste Fluoreszenzsignal ein Autofluoreszenzsignal der ersten Autofluoreszenzkomponente und ein Autofluoreszenzsignal der zweiten Autofluoreszenzkomponente einschließt, wobei die Spektralinformationen jeder der Autofluoreszenzkomponenten abhängig von einer Immobilisierung variieren und wobei das zweite Fluoreszenzsignal ein Fluoreszenzsignal des Fluoreszenzreagenzes einschließt,
eine Segmentierung basierend auf dem getrennten ersten und zweiten Fluoreszenzsignal zum Erkennen eines Bereichs eines Objekts oder Gewebes durchführt, der in den Bildinformationen eingeschlossen ist, und
einen Fixierungszustand der Probe gemäß einem Komponentenverhältnis jedes des Autofluoreszenzsignals der ersten Autofluoreszenzkomponente und des Autofluoreszenzsignals der zweiten Autofluoreszenzkomponente analysiert.

2. Informationsverarbeitungseinrichtung nach Anspruch 1, wobei
die Autofluoreszenzkomponente, die in der Probe eingeschlossen ist, mindestens eines oder mehrere von NADH, d. h. reduziertes Nicotinamid-Adenin-Dinukleotid, FAD, d. h. Flavin-Adenin-Dinukleotid, oder Porphin einschließt.

3. Informationsverarbeitungseinrichtung nach Anspruch 1, wobei
die Informationen bezüglich der Probe ferner Informationen bezüglich einer zu verwendenden Gewebeart, einer Krankheitsart einer Zielperson, einer Eigenschaft einer Zielperson und einer Lebensweise der Zielperson einschließt.

4. Informationsverarbeitungseinrichtung nach Anspruch 1, wobei
der Informationserfassungsabschnitt die Informationen bezüglich der Probe gemäß den Probenidentifikationsinformationen erfasst, die in der Lage sind, die Probe zu identifizieren, und die Informationen bezüglich des Fluoreszenzreagenzes gemäß den Reagenzidentifikationsinformationen erfasst, die in der Lage sind, das Fluoreszenzreagenz zu identifizieren.

5. Informationsverarbeitungseinrichtung nach Anspruch 4, wobei

die Reagenzidentifikationsinformationen ferner in der Lage sind, eine Produktionscharge des Fluoreszenzreagenzes zu identifizieren.

**6.** Informationsverarbeitungseinrichtung nach Anspruch 1, ferner umfassend:
einen Anzeigeabschnitt (140), der Bildinformationen anzeigt, die basierend auf dem ersten Fluoreszenzsignal oder dem zweiten Fluoreszenzsignal erzeugt wurden.

**7.** Informationsverarbeitungseinrichtung nach Anspruch 1, wobei

die Informationen bezüglich des Fluoreszenzreagenzes eine Quantenausbeute, ein Fluoreszenzmarkierungsverhältnis und einen Absorptionsquerschnitt oder einen molaren Absorptionskoeffizienten hinsichtlich des Fluoreszenzreagenzes einschließen; und
der Analyseabschnitt mindestens eines von der Anzahl von Fluoreszenzmolekülen in den Bildinformationen oder der Anzahl von Antikörpern, die an die Fluoreszenzmoleküle unter Verwendung der Bildinformationen gekoppelt sind, aus denen die Autofluoreszenzsignale entfernt werden, und Informationen bezüglich des Fluoreszenzreagenzes berechnet.

**8.** Mikroskopsystem, umfassend:

eine Informationsverarbeitungseinrichtung (100) nach Anspruch 1; und
ein Programm zum Veranlassen der Informationsverarbeitungseinrichtung, eine Steuerfunktion zum Steuern auszuführen, um:

das erste Fluoreszenzsignal zu trennen und die zweite Fluoreszenz zu trennen, um die Segmentierung durchzuführen, und
den Fixierungszustand der Probe zu analysieren.


**Revendications**

**1.** Appareil de traitement d'informations (100), comprenant :

une section d'acquisition d'images qui acquiert des informations d'image d'un échantillon (20) coloré par un réactif de fluorescence (10) ;
une section d'acquisition d'informations (112) qui acquiert un canal de mesure indiquant un premier composant autofluorescent et un second composant autofluorescent inclus dans l'échantillon et des informations spectrales concernant un spectre autofluorescent possédé par chacun du premier composant autofluorescent et du second composant autofluorescent inclus dans l'échantillon et des informations spectrales concernant un spectre de fluorescence détenu par des composants fluorescents inclus dans le réactif de fluorescence ; et
une section d'analyse (131) qui
sépare un premier signal de fluorescence des informations d'image conformément au canal de mesure et des informations spectrales concernant l'échantillon et sépare un second signal de fluorescence des information d'image conformément aux informations spectrales concernant les composants fluorescents inclus dans le réactif de fluorescence, dans lequel le premier signal de fluorescence comporte un signal d'autofluorescence du premier composant autofluorescent et un signal d'autofluorescence du second composant autofluorescent, dans lequel les informations spectrales de chacun des composants autofluorescents varient selon l'immobilisation, et dans lequel le second signal de fluorescence comporte un signal de fluorescence du réactif de fluorescence,
effectue une segmentation, sur la base des premier et second signaux de fluorescence séparés, pour reconnaître une région d'un objet ou d'un tissu inclus dans les informations d'image, et
analyse un état de fixation de l'échantillon conformément à un rapport de composant de chacun du signal d'autofluorescence du premier composant autofluorescent et du signal d'autofluorescence du second composant autofluorescent.

**2.** Appareil de traitement d'informations selon la revendication 1, dans lequel
le composant autofluorescent inclus dans l'échantillon comporte au moins un ou plusieurs parmi NADH, c'est-à-dire nicotine-adénine-dinucléotide hydrogéné, FAD, c'est-à-dire flavine-adénine-dinucléotide, ou porphine.

**3.** Appareil de traitement d'informations selon la revendication 1, dans lequel
les informations concernant l'échantillon comportent en outre des informations concernant un type de tissu à utiliser, un type de maladie d'une cible, un attribut d'une personne cible et une habitude de vie de la personne cible.

**4.** Appareil de traitement d'informations selon la revendication 1, dans lequel
la section d'acquisition d'informations acquiert les informations concernant l'échantillon conformément aux informations d'identification d'échantillon permettant d'identifier l'échantillon et acquiert les informations concernant le réactif de fluorescence conformément aux informations d'identification du réactif permettant d'identifier le réactif de fluorescence.

**5.** Appareil de traitement d'informations selon la revendication 4, dans lequel
les informations d'identification du réactif permettant en outre d'identifier un lot de production du réactif de fluorescence.

**6.** Appareil de traitement d'informations selon la revendication 1, comprenant en outre :
une section d'affichage (140) qui affiche des informations d'image générées sur la base du premier signal de fluorescence ou du second signal de fluorescence.

**7.** Appareil de traitement d'informations selon la revendication 1, dans lequel

les informations concernant le réactif fluorescent comportent un rendement quantique, un rapport de marquage fluorescent et une section transversale d'absorption ou un coefficient d'absorption molaire concernant le réactif fluorescent ; et
la section d'analyse calcule au moins l'un parmi le nombre de molécules de fluorescence dans les informations d'image ou le nombre d'anticorps couplés aux molécules de fluorescence à l'aide des informations d'image à partir desquelles les signaux d'autofluorescence sont supprimés et des informations concernant le réactif de fluorescence.

**8.** Système de microscope comprenant :

un appareil de traitement d'informations (100) selon la revendication 1 ; et
un programme destiné à amener l'appareil de traitement d'informations à exécuter une fonction de commande pour commander de sorte à :

séparer ledit premier signal de fluorescence et séparer ladite seconde fluorescence pour effectuer ladite segmentation, et
analyser ledit état de fixation de l'échantillon.

[Fig. 1]

[Fig. 2]

FIG. 2

# FIG.3

[Fig. 3]

[Fig. 4]

# FIG.4

```
              ┌─────────┐
              │  START  │
              └─────────┘
                   │
                   ▼
┌──────────────────────────────────────┐
│   DETERMINE FLUORESCENT REAGENT       │──── S1000
│           AND SPECIMEN                │
└──────────────────────────────────────┘
                   │
                   ▼
┌──────────────────────────────────────┐
│               STAIN                   │──── S1004
└──────────────────────────────────────┘
                   │
                   ▼
┌──────────────────────────────────────┐
│   IMAGE FLUORESCENTLY STAINED SPECIMEN │──── S1008
└──────────────────────────────────────┘
                   │
                   ▼
┌──────────────────────────────────────┐
│   ACQUIRE REAGENT INFORMATION AND     │──── S1012
│        SPECIMEN INFORMATION           │
└──────────────────────────────────────┘
                   │
                   ▼
                  ( A )
```

[Fig. 5]

# F I G . 5

[Fig. 6]

# FIG.6

<BEFORE AUTOFLUORESCENCE SIGNAL REMOVAL>    <AFTER AUTOFLUORESCENCE SIGNAL REMOVAL>

(A-1)    (B-1)

<STAINED>

(A-2)    (B-2)

<UNSTAINED>

EP 3 827 248 B1

[Fig. 7]

# FIG.7

[Fig. 8A]

# F I G . 8 A

[Fig. 8B]

# F I G . 8 B

[Fig. 9A]

# FIG.9A

[Fig. 9B]

# FIG.9B

[Fig. 10]

[Fig. 11]

# FIG.11

EP 3 827 248 B1

(A1)　　　(A2)　　　(A3)　　　(A4)　　　(A5)　　　(A6)

AUTOFLUORESCENCE IMAGE

(B1)　　　(B2)　　　(B3)　　　(B4)　　　(B5)
DAPI　　　CD4/AF488　CD3/AF555　Ki-67/AF568　PDL-1/AF594

FLUORESCENCE IMAGE

(B6)　　　(B7)　　　(B8)
PD-1/AF647　CD8/AF680　CD68/AF700

[Fig. 12]

# F I G . 1 2

(A1)

STAINING: TONSIL
AUTOFLUORESCENCE: TONSIL
CD4/AF488

(B1)

STAINING: TONSIL
AUTOFLUORESCENCE: LUNG CANCER
CD4/AF488

(A2)

(B2)

EP 3 827 248 B1

# FIG.13

[Fig. 13]

(A1)

STAINING: TONSIL
AUTOFLUORESCENCE: TONSIL
PDL-1/AF594

X1 ——————————— X2

(B1)

STAINING: TONSIL
AUTOFLUORESCENCE: LUNG CANCER
PDL-1/AF594

X1 ——————————— X2

(A2)

(B2)

EP 3 827 248 B1

[Fig. 14]

# FIG.14

[Fig. 15]

# FIG.15

[Fig. 16]

# FIG.16

[Fig. 17]

# FIG.17

# FIG.18

[Fig. 19]

# FIG.19

WAVELENGTH (nm)

[Fig. 20]

# FIG.20

WAVELENGTH (nm)

EP 3 827 248 B1

FIG.21

[Fig. 22]

# FIG.22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20160035100 A1
- US 20080294032 A1
- US 20140376087 A
- WO 2017072320 A1

### Non-patent literature cited in the description

- **ZHANG W. et al.** Fully automated 5-plex fluorescent immunohistochemistry with tyramide signal amplification and same species antibodies. *Laboratory Investigation,* 15 May 2017, http://www.nature.com/articles/labinvest201737

- **MANSFIELD et al.** Autofluorescence removal, multiplexing, and automated analysis methods for in-vivo fluorescence imaging. *Journal of biomedical Optics,* 01 January 2005, vol. 10 (4), ISSN 1083-3668, 041207
- **KNIGHT ; BILLINTON.** Distinguishing GFP from Cellular Autofluorescence. *Biophotonics International,* 01 September 2001, vol. 8, 1-6